# EUROPEAN PATENT SPECIFICATION

(11) **EP 2 571 505 B1**
(45) Date of publication and mention of the grant of the patent: **06.07.2016**
(21) Application number: 11722320.6
(22) Date of filing: 18.05.2011
(51) Int. Cl.: A61K 31/573, A61P 5/44, A61K 9/20

(54) **POSOLOGY AND ADMINISTRATION OF GLUCOCORTICOID BASED COMPOSITIONS**
DOSIERUNG UND VERABREICHUNG VON ZUSAMMENSETZUNGEN AUF GLUKOKORTIKOIDBASIS
POSOLOGIE ET ADMINISTRATION DE COMPOSITIONS À BASE DE GLUCOCORTICOÏDE

(30) Priority: 20.05.2010 DK 201000442
(43) Date of publication of application: 27.03.2013
(73) Proprietor: Shire Viropharma Incorporated, Lexington MA 02421 (US)
(72) Inventor: HEDNER, Thomas, S-520 10 Gällstad (SE); SIMONSSON, Ulrika, Sigrida, Helena, S-443 72 Gräbo (SE); JOHANNSSON, Gudmundur, S-302 40 Halmstad (SE); LENNERNÄS, Hans, S-756 52 Uppsala (SE); SKRTIC, Stanko, S-431 36 Mölndal (SE)
(74) Representative: Goodfellow, Hugh Robin
(86) International application number: PCT/EP2011/002466
(87) International publication number: WO 2011/144327

(56) References cited:
- WO-A-03/015793
- WO-A-2005/102271
- WO-A1-2010/115615
- DEBONO MIGUEL ET AL: "Inadequacies of glucocorticoid replacement and improvements by physiological circadian therapy.", EUROPEAN JOURNAL OF ENDOCRINOLOGY / EUROPEAN FEDERATION OF ENDOCRINE SOCIETIES MAY 2009, vol. 160, no. 5, May 2009 (2009-05), pages 719-729, XP002545366, ISSN: 1479-683X
- JOHANNSSON GUDMUNDUR ET AL: "Improving glucocorticoid replacement therapy using a novel modified-release hydrocortisone tablet: a pharmacokinetic study.", EUROPEAN JOURNAL OF ENDOCRINOLOGY / EUROPEAN FEDERATION OF ENDOCRINE SOCIETIES JUL 2009 LNKD- PUBMED:19383806, vol. 161, no. 1, July 2009 (2009-07), pages 119-130, XP002609594, ISSN: 1479-683X
- JOHANNSSON GUDMUNDUR ET AL: "The metabolic impact of improving diurnal cortisol exposure in patients with adrenal insufficiency", ENDOCRINE JOURNAL, vol. 57, no. Suppl. 2, March 2010 (2010-03), page S307, XP008129174, & 14TH INTERNATIONAL CONGRESS OF ENDOCRINOLOGY ICE2010; KYOTO, JAPAN; MARCH 26 -30, 2010 ISSN: 0918-8959
- DEBONO M ET AL: "Novel strategies for hydrocortisone replacement.", BEST PRACTICE & RESEARCH. CLINICAL ENDOCRINOLOGY & METABOLISM APR 2009, vol. 23, no. 2, April 2009 (2009-04), pages 221-232, XP002545367, ISSN: 1532-1908
- XU J ET AL: "Assessment of the impact of dosing time on the pharmacokinetics/ pharmacodynamics of prednisolone", AAPS JOURNAL 200806 US, vol. 10, no. 2, June 2008 (2008-06), pages 331-341, XP008111796, ISSN: 1550-7416
- LENNERNAS H ET AL: "Replacement therapy of oral hydrocortisone in adrenal insufficiency: The influence of gastrointestinal factors", EXPERT OPINION ON DRUG METABOLISM AND TOXICOLOGY 200806 GB LNKD- DOI:10.1517/17425255.4.6.749, vol. 4, no. 6, June 2008 (2008-06), pages 749-758, XP008129168, ISSN: 1742-5255

## Description

### Field of the invention

The present invention relates to glucocorticoid based compositions for use in glucocorticoid replacement therapies according to claim 1 enabling an objectively based regimen for administration enabling correct individual dosing of glucocorticoids resulting in an optimised individual replacement therapy and thus an improved long-term outcome for patients with temporary or chronic adrenal insufficiency.

### Introduction

Glucocorticoids (GC) are important steroids for intermediary metabolism, immune function, musculoskeletal function, connective tissue and brain function. GC deficiency occurs in adrenal insufficiency (Al) which can be primary (Addison's disease), secondary (central) due to hypopituitarism.

Standard therapies in glucocorticoid replacement therapy include administration thrice daily with e.g. hydrocortisone in doses of 5-20mg with a total daily dose of 15-60 mg of hydrocortisone. The exact dose to be administered is usually based on the individual physicians own experience starting from a low dose of glucocorticoid and judging from the subjective response of the patient gradually increase the dose in case the subject shows insufficient response (in case of glucocorticoid deficiency) or decrease the dose if the subject shows symptoms of glucocorticoid excess. However, this approach has several disadvantages, i.a. problem of over or under dosing can easily follow as a result thereof which in turn may be due to individual variability in the sensitivity towards a certain dose of certain glucocorticoid. The known side effects of under or overtreatment with glucocorticoids are e.g. immunosuppression, hyperglycemia due to increased gluconeogenesis, insulin resistance and impaired glucose tolerance ("steroid diabetes"), increased skin fragility (easy bruising), negative calcium balance due to reduced intestinal calcium absorption, steroid-induced osteoporosis (osteoporosis, osteonecrosis, higher fracture risk, slower fracture repair), weight gain due to increased visceral and truncal fat deposition (central obesity) and appetite stimulation, muscle breakdown (proteolysis), weakness; reduced muscle mass and repair, and dilation of small blood vessels in skin, anovulation, irregularity of menstrual periods, growth failure, pubertal delay increased plasma amino acids, increased urea formation; negative nitrogen balance, excitatory effect on central nervous system (euphoria, psychosis), glaucoma, increased cranial pressure, cataracts. In patients with chronic adrenal insufficiency under treatment may even fatal.

Large dosages of hydrocortisone can cause elevation of blood pressure, salt and water retention, and increased excretion of potassium. Long term treatment with higher than physiological hydrocortisone doses can lead to clinical features resembling Cushing syndrome with increased body fat, abdominal obesity, hypertension and diabetes, and thus an increased risk of cardiovascular morbidity and mortality.

Old age and low body mass index are known risk factors for common side effects of pharmacological doses of glucocorticoids such as osteoporosis, thinning of skin, diabetes mellitus, hypertension and increased susceptibility to infections.

All glucocorticoids increase calcium excretion and reduce the bone-remodelling rate. Patients with adrenal insufficiency on long-term glucocorticoid replacement therapy have been found to have reduced bone mineral density, but fracture frequency has not been studied.

Prolonged use of high doses of glucocorticoids may produce posterior subcapsular cataracts, and glaucoma with possible damage to the optic nerves. Such effects have not been reported in patients receiving replacement therapy with glucocorticoids in doses used in adrenal insufficiency.

Psychiatric adverse reactions may occur with systemic glucocorticoids. This may occur during commencement of treatment and during dose adjustments. Risks may be higher when high doses are given. Most reactions resolve after dose reduction, although specific treatment may be necessary.

Treatment of primary adrenal insufficiency often warrants addition of a mineralocorticoid.

Thus adequate assessment of patients on glucocorticoid replacement therapy is of great importance to avoid the consequences of under or over treatment, but currently no simple test or method exists to objectively make such an assessment.

A recent report (Mah, P. M., et al., Clinical Endocrinology, 2004, 61, 367-375) states that a recommended dosing of thrice daily using conventional hydrocortisone tablets before food intake monitored with a single serum cortisol measurement using a weight based nomogram is the best treatment method. However, the total daily doses presented in Mah et al., are markedly lower than in present invention and consequently under treatment may result. Moreover, the study above doesn't take into account the residual endogenous cortisol secretion that is commonly present in secondary adrenal insufficiency and also to some extent in some patients with primary adrenal insufficiency.

Another report (Debono , M., et al., best Practise & Res. Clin. Endocrin. & Metabolism, 2009, 23, 221-232) suggest a weight based dosing of 0.12 mg/kg of hydrocortisone in the morning in a thrice daily dosage regimen according to the same nomogram as in Mah et al.

JOHANNSSON GUDMUNDUR ET AL: "Improving glucocorticoid replacement therapy using a novel modified-release hydrocortisone tablet: a pharmacokinetic study.", EUROPEAN JOURNAL OF ENDOCRINOLOGY / EUROPEAN FEDERATION OF ENDOCRINE SOCIETIES JUL 2009 LNKD- PUBMED:19383806, vol. 161, no. 1, July 2009 (2009-07), pages 119-130, ISSN: 1479-683X teaches a glucocorticoid formulation with combined immediate and extended release characteristics containing 20 mg of hydrocortisone (extended release core surrounded by immediate release coating from Duocort = applicant). The tablet was administered once daily in the morning. The study is a pharmacokinetic study in healthy volunteers which aims at developing a treatment for cortisol replacement therapy.

Ross, R., et al., (WO 2003/015793) discuss administration thrice daily according to a weight based nomogram. However, the recommended dosage according to Ross et al., results in lower total daily doses than present invention and may thus result in under treatment.

### Description of the invention

Subject-matter which is not encompassed by the scope of the claims does not form part of the presently claimed invention.

As mentioned above the standard approach of finding the appropriate dose in replacement treatments is to use the lowest possible maintenance dosage. If the patient shows clinical symptoms or signs of glucocorticoid deficiency or glucocorticoid excess, the dosage should be increased or decreased in small steps until a satisfactory response is observed. Standard therapies include a twice-α-day or thrice-α-day administration of glucocorticoids such as hydrocortisone or cortisone acetate. Such therapies are not optimal as over or under treatment may result (vide supra). Patient compliance may be compromised and the natural circadian cortisol rhythm may be poorly mimicked, all resulting in sub-optimal treatment. Consequently, there is a need for a more precise tool when administering glucocorticoids which should ideally avoid over or under treatment, i.e. avoid administering too low doses resulting in under treatment or administering to high doses and thereby exposing the patient to unnecessarily high doses of glucocorticoids.

The inventors have found that by dosing the administered glucocorticoid according to a nomogram which is either weight based or pharmacokinetically based an individualised treatment will result and as a consequence thereof achieve a more physiological glucocorticoid exposure. The method thus represents a more objective approach in finding out the dose to be administered that take into account the individual response/sensitivity to a certain dose of a glucocorticoid and in that way result in a more reliable, informed and objective, individualised and patient secure treatment avoiding under or overtreatment.

Thus, patients receiving conventional oral hydrocortisone either twice or thrice daily can be switched to a glucocorticoid composition according to the invention which encompasses administration once daily which may be once daily in the morning and may further be once daily in the morning before intake of the first meal. However when switching from conventional therapy to a therapy according to the invention it is important to recognise that it is usually not a straightforward switching from one composition in the conventional therapy form and use the identical daily dose of glucocorticoid in the composition according to present invention. The inventors have found that by correlating the weight of the subject or the pharmacokinetic response in the subject a nomographic correlation can be used in finding the correct individual dose to be administered resulting in an optimised individual replacement therapy and thus improve the long-term outcome of patients with adrenal insufficiency. Moreover, if the a *priori* administration regimen using the weight based nomogram is found to be clinically insufficient the dosage regimen may be followed by administration using the *a posteriori* dosage regimen suing the PK nomogram

The inventors of present invention has found that by making a dose individualisation when administering a composition according to the invention, a markedly improved treatment regimen is observed, wherein the administration regimen using the composition according to the invention results in a optimal treatment, hence avoiding under or over exposure of glucocorticoids.

If certain covariates (e.g., age, body weight) are known to affect pharmacokinetics or pharmakodynamics, these could be used to individualise the dose beforehand, so called *a priori* dose adaption. If a dose is derived based on observation of the drug response, this is called *a posteriori* dose adaption. Dose adjustment based on measurement of a drug characteristic such as its serum concentration, rather than using a clinical endpoint or biomarker is known as therapeutic drug monitoring (TDM). Both *a priori* dose individualisation and *a posteriori* dose individualisation often use nomograms where the clinicians easily can depict the recommended dose based on a covariate or measured drug concentration. *A priori* dose individualisation using an easily observed or measured demographic variable would be useful in the treatment of adrenal insufficiency if such a covariate is found to be a good predictor of oral clearance and thereby exposure. A high in-between patient variability in oral clearance, i.e. exposure, after correcting for covariate relationship, would indicate the need for *a posteriori* dose individualisation where the dose recommendation could be made based on measurement of serum concentration of cortisol. Serum cortisol determination is commonly available in routine biochemical analysis in hospitals and would be of great value for most patients during dose titration and monitoring particularly in situations where clinical symptoms and signs are unreliable. *A posteriori* dose individualisation would be of special importance in patients with residual endogenous cortisol production such as in secondary adrenal insufficiency and in some primary adrenal insufficiency patients. For this purpose, the inventors of present invention has developed a method for both *a priori* dosing (weight based nomogram) as well as a *posteriori* dosing (pharmacokinetic nomogram), which may optionally be combined in the sense that if it is found that administration according to the weight based nomogram results in a less than optimal clinical response, the pharmacokinetic nomogram can be used.

### Weight nomogram

Oral clearance of the glucocorticoid according to present invention is dependent on body weight; therefore, a target dose using a weight nomogram can be used guided by clinical status and response to therapy. The clinical exposure target of the weight nomogram is based on an average 24h cortisol exposure in healthy adults. The weight based nomogram may reduce the risk of patients receiving too high doses of hydrocortisone. The inventors of present invention have surprisingly found a relationship between oral clearance, the individual predictor for exposure, and body weight which has formed the basis for the development of the weight nomogram as seen below (Table 1), where the dose denotes the dose of hydrocortisone or hydrocortisone equivalent to be administered

**Table 1**

| Hydrocortisone Dose (mg) | Body weight (kg) |
|---|---|
| 15 | 55-59 |
| 20 | 60-69 |
| 25 | 70-79 |
| 30 | 80-84 |
| 35 | 85-89 |
| 40 | ≥90 |

It is further envisaged that in subject having a body weight of about 50-54 kg a dose of about 10 mg may be appropriate. Furthermore it is also envisaged that when a subject has a body weight of about 45-49 kg a dose of about 5 mg may be appropriate or alternatively a dose of from about 0.5-4 mg may be appropriate. The weight based nomogram is easily applied by weighing the patient and thereafter administering the dose as indicated in the weight based nomogram above. For example, if a subject weighs between 70-79 kg, a dose of 25 mg of hydrocortisone in a composition according to the invention should be administered. In some instances where the weight based nomogram may be unsuitable is when the individual clinical response is found to be insufficient. In these instances it may be suitable to shift to dosages based upon pharmacokinetic nomogram. Instances where this may occur are e.g. subjects suffering from obesity or pregnant subjects.

### Pharmacokinetic nomogram

The between-patient variability in oral clearance is around 23%. This is judged to be a moderate to high in between patient variability. This means that when all other predictors of variability in exposure have been taken into account (i.e. dose and body weight), there is still a fairly high variability between patients. This formed the basis for development of the pharmacokinetic nomogram. A nomogram for dose individualisation based on serum concentration can be used in patients with inadequate clinical response or in patients with some residual adrenal function or when lower replacement doses may be needed. The clinical exposure target of the pharmacokinetic nomogram is based on an average 24h cortisol exposure in healthy adults. The difference in serum cortisol concentration before and 6 hours after post-dosing was found to be the best input into the pharmacokinetic (PK) nomogram. The PK nomogram is a more precise dosing tool compared to the weight nomogram as it takes into account endogenous cortisol levels and unexplained between-patient variability in cortisol clearance. The pharmacokinetic nomogram below (Table 2) indicates the recommended once daily oral dose based on the derived C_{cortisol} value.

**Table 2**

| | C₀ₕ ≤150 nM | 150< C₀ₕ ≤250 nM | 250< C₀ₕ ≤350 nM |
|---|---|---|---|
| C_{cortisol} (nM) | Dose (mg) | Dose (mg) | Dose (mg) |
| ≥198 | 15 | 10 | 5 |
| 197-161 | 20 | 10 | 5 |
| 160-141 | 25 | 15 | 10 |
| 140-115 | 30 | 15 | 10 |
| 114-97 | 35 | 15 | 10 |
| <97 | 40 | 15 | 10 |

C_{cortisol} denotes the cortisol concentration in the blood between 6 hours post-dose (C₆ₕ) and pre-dose (C₀ₕ) and is thus calculated as the numerical difference in cortisol serum concentrations in the subject just before the dose is administered and 6 hours after the administered dose. Two blood samples need to be drawn to derive the dose from the PK nomogram in order to account for putative pre-dose cortisol levels. Therefore, the procedure for deriving an individual dose based on the PK nomogram is as follows:
1. A blood sample is taken pre-dose from the subject in fasted state
2. An oral test dose of 20 mg of hydrocortisone or hydrocortisone equivalent in a composition according to the invention is administered to the subject. This should be the first dose of hydrocortisone or hydrocortisone equivalent administered during that particular day.
3. Exactly 6 hours post-dose, a second blood sample is withdrawn from the subject.
4. The serum cortisol concentration is determined in the two samples as stated under points 1 and 3 above by an immunoassay method.
5. The difference in serum cortisol concentration (C_{cortisol}= C₆ₕ -C₀ₕ) between 6 hours post-dose (C₆ₕ) and pre-dose (C₀ₕ) is derived
6. The individual of composition dose is derived based on the derived C_{cortisol} and C₀ₕ values and the PK nomogram above.

Consequently, by measuring the C_{cortisol} and the C₀ₕ, the appropriate dose to be administered to the patient is readily found in the table above. For example, if the C_{cortisol} is found to be between 197-161 nM and the C₀ₕ is found to be within the interval of 150< C₀ₕ ≤250 nM, a dose of 10 mg of hydrocortisone in a composition according to the invention should be administered.

The nomograms according to the invention are intended to be used in the dosage regimens using the composition as disclosed herein with the specified release pattern between the immediate release and extended release. However, the dosage regimen according to the invention using the nomograms as disclosed herein may also be used with similar glucocorticoid compositions having similar release patterns of glucocorticoids as disclosed herein.

Monitoring of serum cortisol is both a measurement of drug exposure and of the pharmacodynamic response since hydrocortisone replacement therapy directly replaces the deficient hormone cortisol. As such, serum cortisol sampling should be a more accurate and precise tool as compared to many situations where pharmakokinteics in therapeutic drug monitoring replaces monitoring of a biomarker or a clinical outcome. In addition, a pharmacokinetic nomogram based on serum cortisol determination is of special need in patients with secondary adrenal insufficiency. This patient population includes a higher proportion of patients with some remaining endogenous cortisol production and therefore similar doses to those in Addison's disease may not be needed (Agha et al. 2004). A pharmacokinetic nomogram would provide a tool to tailor the individual dose since the endogenous cortisol level could be accounted for by subtracting the post-dose concentration from a pre-dose concentration and the concentration difference may be used as input to the pharmacokinetic nomogram in order to derive the individual dose. Due to the lack of a biomarker for the clinical end-points combined with cortisol pharmacokinetics being associated to the pharmakodynamics, the clinical target for the nomograms should be a cortisol exposure equivalent to that observed in healthy volunteers.

The use of these nomograms is suitable in subjects of an age of 11 years or more.

### Use in intercurrent/transient illness

During intercurrent illness there should be high awareness of the risk of developing acute adrenal insufficiency. In such situations, oral administration of hydrocortisone must be replaced with parenteral treatment and an increase in dose is immediately required. Parenteral administration of hydrocortisone is warranted during transient illness episodes such as infections, in particular gastroenteritis, high fever of any aetiology or extensive physical stress, such as surgery under general anaesthesia.

In situations when parenteral administration of hydrocortisone is not required, such as low grade infection, fever of any aetiology or minor surgical procedures, the normal oral replacement dose must be increased temporarily; the daily dose of Plenadren should be given twice or thrice daily with 8 ± 2 hours intervals. Once the intercurrent illness episode is over, patients can return to the normal maintenance dose of Plenadren..

The daily total dose of hydrocortisone in e.g. Addison's disease patients is a maintenance dose that is not enough during a stressful event. The rescue dose needed for the patient to cope with a stressful event such as stress, fever and other intercurrent illness situations is very individual and based on the experience of the patients initially guided by the treating physician. Dosing recommendations are necessary for these situations. Thus during intercurrent illness, dosage regimens of doubling the maintenance dose from once daily to twice daily may be warranted. It would be useful to investigate additional dosing regimens with different dosing intervals for the second dose as well as tripling the dose. This would be impossible to study without inclusion of a large number of patients which would be practically difficult due to the limited number of diseased patients and due the fairly low number of intercurrent events per individual and year (White et al 2010). Pharmacometric simulations could therefore provide additional information needed to suggest intercurrent illness regimens. Moreover, previous recommendations have assumed that doubling of the dose will lead to doubling of cortisol exposure therefore not taking into account the non-linear bioavailability hydrocortisone during higher oral doses.

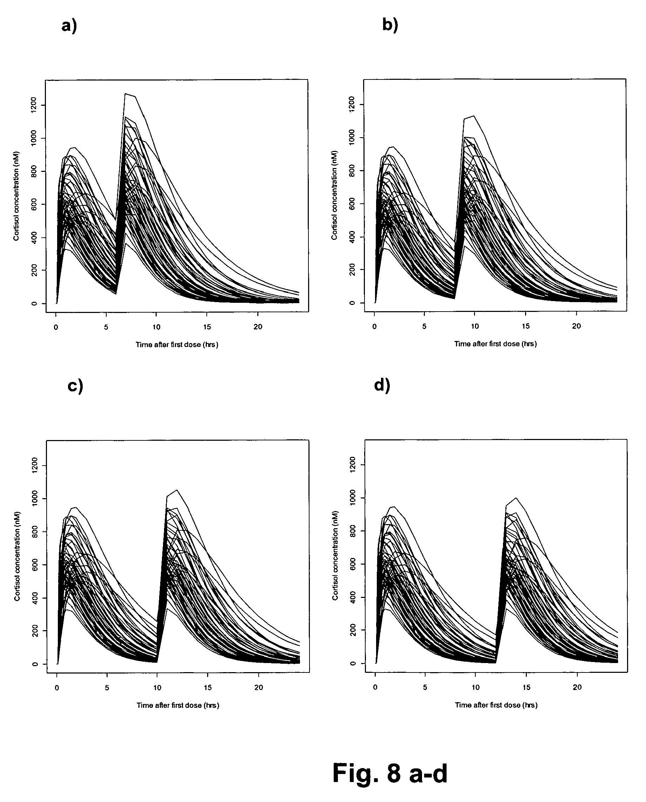

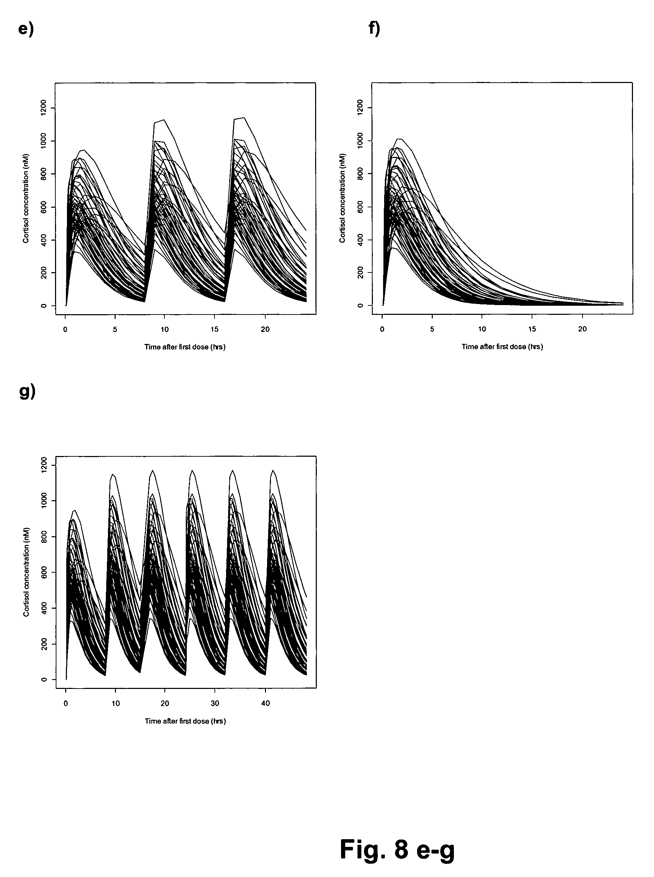

The inventors have found that simulations of intercurrent illness regimens shows that giving an additional dose at 8±2 hours after the first morning dose resulted in slightly higher peak concentrations for the second dose but still fairly low concentrations during the night (Fig. 8a-c). This regimen is therefore recommended for milder clinical conditions, e.g., low-grade infection. Giving three daily doses of hydrocortisone in a composition according to present invention with 8 hours intervals resulted in no accumulation after the third daily dose compared to the second and with elevated cortisol levels during the night as well (Fig. 8d). This regimen is therefore recommended in more serious conditions. Increasing the oral dose but keeping the once daily administration regimen does not provide sufficient cortisol coverage in intercurrent illness conditions (Fig. 8e). The simulations of intercurrent illness was made with 30 mg of hydrocortisone in a composition but the conclusions are valid for the other dose levels as well since the accumulation and time to steady state is only dependent on the terminal half-life which is dose-independent.

Consequently, during an intercurrent illness there should be high awareness of the risk of developing acute adrenal insufficiency. High parental doses of glucocorticoids may then be warranted. During acute adrenal insufficiency parenteral administration of hydrocortisone in high doses, together with saline infusion, may be given with or without simultaneous administration of hydrocortisone in a composition according to the invention.

During transient illnesses such as infection, fever of any aetiology or extensive physical stress such as surgery, the replacement dose may be increased temporarily, such as increased by e.g. at least 5mg or more, such as e.g. at least 10 mg or more, such as e.g. at least 15 mg or more, such as e.g. at least 20 mg or more, such as e.g. at least 25 mg or more, such as e.g. at least 30 mg or more, such as e.g. at least 35 mg or more, such as e.g. at least 40 mg or more, such as e.g. at least 45 mg or more, such as e.g. at least 50mg or more, such as e.g. at least 55mg or more, such as e.g. at least 60mg or more, such as e.g. at least 65mg or more, such as e.g. at least70mg or more, such as e.g. at least 75mg or more, such as e.g. at least 80mg or more, such as e.g. at least 90mg or more, such as e.g. at least 100mg or more, such as e.g. at least 150mg or more, such as e.g. at least 200mg or more, such as e.g. at least 300mg or more.

### Use in replacement therapies for chronic adrenocortical insufficiency

The onset of adrenocortical insufficiency may vary from insidious to an acute life-threatening situation with severe salt and water deficit, which leads to shock and death if not treated adequately. Frequently reported symptoms associated with more insidious adrenocortical insufficiency are asthenia, weakness, lethargy, easy fatigability, nervousness, irritability, apathy, dizziness, headache, myalgia, anorexia, weight loss, nausea, vomiting, and diarrhoea. A recent review by Arit et al. (Lancet (2003) 361, 1881-1893) *inter alia* describes conditions leading to adrenal insufficiency and is hereby incorporated by reference. Three general types of adrenocortical insufficiency can be discerned. Primary adrenocortical insufficiency is usually referred to as Addison's disease. In this disorder, the adrenal cortex is affected meaning that the function of the three hormone systems produced in the adrenal cortex is impaired. The consequence of Addison's disease is therefore insufficient production and secretion of cortisol, adrenal androgens and mineralocorticoids (aldosterone).

Secondary or central adrenocortical insufficiency is mainly caused by tumours in the hypothalamic-pituitary area. The problem and the treatment considerations of secondary glucocorticoid deficiency are, however, similar to those in patients with primary adrenal failure.

Tertiary adrenal insufficiency is probably the most common cause of glucocorticoid deficiency. It is a result of long term, high dose glucocorticoid therapy as a part of treatment in patients with pulmonary diseases, autoimmune and inflammatory diseases and in the treatment of various malignancies, which results in the suppression of endogenous secretion of adrenal glucocorticoids. Tertiary adrenal insufficiency may last from a few weeks to a year.

In most cases of primary and secondary adrenal insufficiency replacement therapy with glucocorticoids is a life long treatment. The aim of glucocorticoid replacement therapy is to mimic the circadian serum cortisol profile, respond to the increased cortisol need during physical and psychological stimuli and obtain normal well-being, metabolism and long-term outcome. Both during childhood and adulthood, under-treatment can lead to malaise, postural hypotension, poor response to stress and electrolyte disturbances and even acute adrenal crisis. In childhood, an appropriate replacement dose of glucocorticoids is crucial to avoid growth suppression and reduced final height potential that are associated with glucocorticoid excess. In adults, excessive glucocorticoid replacement may induce glucose intolerance, abdominal obesity, hypertension, protein catabolism and osteoporosis.

Accordingly, the invention relates to glucocortocoid based compositions for use in glucocorticoid replacement therapy by administration according to a nomogram according to claim 1. The glucocorticoid deficiency may thus be due to chronic illness or it may be due to any temporary condition such as e.g. injury or surgery which may result in an impaired ability to up-hold a circadian plasma concentration profile of cortisol of a healthy subject. In any such instance the compositions for use according to the invention is intended. The invention further enables a method of treating a subject in need of a replacement therapy in which the compositions according to the invention are administered according to a nomogram that is either weight based or PK-based.

It is thus to be clearly understood that depending on the clinical situation, the compositions according to the invention may be supplemented by administering additional doses of glucocortiocoids e.g supplemented with administration of a conventional hyrocortison etbalet or parenterally administering an injection of hydrocortisone. This may be such as e.g. twice daily or e.g. thrice daily or e.g. 4 times daily or e.g. 5 times daily or e.g. 6 times daily. It is also to be understood that the time interval between administered doses may be variable. The time interval between the first administration and the second may be e.g. about 2 hours or more, such as e.g. about 3 hours or more, such as e.g. about 4 hours or more, such as e.g. about 5 hours or more, such as e.g. about 6 hours or more, such as e.g. about 7 hours or more, such as e.g. about 8 hours or more, such as e.g. about 9 hours or more, such as e.g. about 10 hours or more, such as e.g. about 11 hours or more, such as e.g. about 12 hours or more. Independently of each other, the time interval between any consecutive administrations may e.g. about 2 hours or more, such as e.g. about 3 hours or more, such as e.g. about 4 hours or more, such as e.g. about 5 hours or more, such as e.g. about 6 hours or more, such as e.g. about 7 hours or more, such as e.g. about 8 hours or more, such as e.g. about 9 hours or more, such as e.g. about 10 hours or more, such as e.g. about 11 hours or more, such as e.g. about 12 hours or more. Furthermore, the total daily dose of glucocorticoids according to the invention are e.g. about 1 mg or about 5 mg or about 10 mg or about 15 mg or about 20 mg or about 25 mg or about 30 mg or about 40 mg or about 50 mg or about 60 mg or about 70 mg or about 80 mg or about 90 mg or about 100 mg or about 150 mg or about 200 mg. Morevover, it is to be clearly understood that a glucocorticoid in a composition according to the invention may be administered as a combination of different doses so as to make up the total final dose of glucocorticoid to be administered. For example, if a dose of 30 mg of glucocorticoid is to be administered, two compositions comprising 5 mg of the glucocorticoid and one composition comprising 20 mg of glucocorticoid is administered.

### Definitions

The term *"standard therapy"* as used in the present context is intended to denote a therapy involving oral administration three times daily of a glucocorticoid-containing composition, wherein the composition is a conventional tablet composition with immediate release of the glucocorticoid. Accordingly, the term "standard therapy" does not include treatment with e.g. a controlled release composition or treatment with e.g. a combination of a controlled release composition and an immediate release composition. With hydrocortisone as an example, standard therapy is suitable carried out using Cortef® (Pfizer), Hydrocorton ® (Merck), Hydrocortisone (generic e.g. MSD, Nycomed, Teva, Auden McKenzie).

The term "*glucocorticoid*" means any steroid or steroid analog the can bind and activate the glucocorticoid receptor both through its genomic and non-genomic pathway.

The term "*mineralocorticoid*" is intended to mean a class of steroid hormones characterised by their similarity to aldosterone and their influence on salt and water balance acting primarily through the mineralocorticoid receptor.

The term *"circadian plasma concentration of cortisol"* means that the cortisol concentration during day and night follows that of a healthy subject measured using an immunoassay in either serum or plasma.

| Clock time | Plasma concentration of cortisol |
|---|---|
| 6 - 6.30 am | >200 nmol/L |
| 6 am - 10 am | 200 - 500 nmol/L |
| 6 pm - 12 pm | 50 - 200 nmol/L |
| 0 am - 6 am | < 50 nmol/L |

The term "*mimics*" means that the specified regimen mimics the circadian secretion pattern of cortisol and more specifically replace the daytime cortisol secretion; high morning plasma concentrations with slowly reduction in the plasma concentration throughout the day and with low nighttime plasma cortisol concentration. Accordingly, the term "mimics" has its ordinary meaning that is to resemble, simulate, approximate, follow or impersonate, but not replicate exactly or precisely. See also the discussion above.

The term "*once daily*" means that administration takes place only one time during the day. The administration may include more than one composition and more than one administration route. Preferably, the one or more oral dosage forms are administered (such as one or more single-unit tablet formulation). The daily administration time is preferably at the same time of the day (within 0.5 - 1.5 hours variation).

The term "*subject*" means a mammalian subject including dogs, cats and horses. Preferred subjects are humans.

The term "*subject with insufficient adrenal capacity*" is intended to mean a subject that due to chronic or temporary disease or condition is unable to endogenously produce and up-hold a circadian plasma concentration profile of cortisol found in a healthy subject.

The term "immediate release" is generally used in accordance with the regulatory term for conventional, ordinary or plan tablets. The regulatory term for all release-controlled or release-modified tablets is "modified release". In those cases where a distinguish is made between tablets, which are conventional tablets without any release-modfying characteristics and those, which may have enhanced release characteristic, the conventional tablets are without enhanced, controlled or modified release characteristics.

### The term "cortisone" includes "cortisone acetate"

The term "C₀ₕ" is intended to mean the cortisol serum concentration in a subject observed just prior to receiving a glucocorticoid composition. The term "C₆ₕ" is intended to mean the observed cortisol serum concentration (in nM) in a subject 6 hours after receiving a 20 mg glucocorticod composition according to the invention.

The term "C_{cortisol}" is intended to mean the difference in the values between C₆ₕ and C₀ₕ given in nM.

The term "clinical exposure target" is intended to mean the healthy normal cortisol AUC0-24h population average More stringently this is defined as AUC_{target} = [Fᵣₑₗ.dose]/TV(CL/F), wherein the AUC_{target} is the clinical exposure target and Fᵣₑₗ is the model predicted relative bioavailability (F) taking into account the nonlinear relationship between dose and F. TV(CL/F) is the model predicted typical oral clearance including the covariate relationships.

The term "test dose" is intended to mean a tablet according to the invention containing 20 mg in total of hydrocortisone.

Under the heading "Pharmaceutical compositions" more details are given with respect to compositions that have the desired properties with respect to mimicking the circadian rhythm of cortisol after administration.

### Active substance

In the present context, the term "glucocorticoid" or "glucocorticosteroid" is intended to denote a therapeutically, prophylactically and/or diagnostically active glucocorticoid or a glucocorticoid that has physiologic effect. The term is intended to include the glucocorticoid in any suitable form such as e.g. a pharmaceutically acceptable salt, complex, solvate, ester, active metabolites or prodrug thereof of in any physical form such as, e.g., in the form of crystals, amorphous or a polymorphous form or, if relevant, in any stereoisomer form including any enantiomeric or racemic form, or a combination of any of the above. The glucocorticoid may be a synthetic glucocorticoid.

The one or more glucocorticoids contained in a composition according to the invention is selected from the group consisting of hydrocortisone, cortisone, prednisolone, prednisone, methylprednisone, triamcinolone, paramethasone, betamethasone, dexamethasone, fludrocortisone, budesonide, fluticasone, cortisone acetate, deoxycorticosterone, aldosterone and beclometasone including pharmaceutically acceptable esters, salts and complexes thereof.

As mentioned above, it is important to achieve a plasma cortisol concentration-time profile that mimics that of a healthy subject. Accordingly, the one or more glucocorticoids may be presented in a suitable delivery system such as a dosage form. Moreover, a part (first part) of the glucocorticoid must be released from the delivery system faster than another part (second part) in order to enable a fast appearance of the glucocorticoid in the plasma (relating to the first part) followed by a maintenance dose (extended release of the second part). The first and the second part may be presented in the same formulation or in separate formulations. In a preferred formulation, they are presented in the same formulation, notably a single-unit formulation. Moreover, if they are presented in separate formulations, the first and second formulation may be designed to be administered by the same or different administration route. These aspects are discussed further below.

The one or more glucocorticoids of the first and the second part may be the same glucocorticoid or a mixture of the same glucocorticoids. Normally, this is the case as it is easy from a manufacturing point of view in those cases where both the first and the second part are parts of the same dosage form (e.g. the first and second part are contained in a tablet and the first part is provided as a coating or as a separate layer on a core containing the second part). However, in those cases where the first and second part are not part of the same dosage form (e.g. the first part is an effervescent tablet and the second part is in the form of an extended release tablet) or in those cases where an improved therapeutic result is expected when different glucocorticoids are employed, the one or more glucocorticoids of the first and the second part are different glucocorticoids or a mixture of different glucocorticoids.

As the first part of the glucocorticoid is intended for immediate release, the release and/or absorption may take place already in the oral cavity in the case the composition is administered orally. In such cases, the glucocorticoid of choice for the first part may not be hydrocortisone (as such) or cortisone as these two active substances have a bitter taste. However, these substances may be employed provided that a sufficient taste masking is obtained. In the paragraph relating to "Pharmaceutically acceptable excipients" taste-masking is discussed in more detail. Accordingly, the one or more glucocorticoids of the first part may have an acceptable taste, may be tasteless or may be effectively taste-masked.

Examples of the one or more glucocorticoids of the first part (as discussed above) are synthetic glucocorticoids such as, e.g., hydrocortisone 21-succinate, prednisolone, prednisone, methylprednisone, triamcinolone, paramethasone, betamethasone, dexamethasone, fludrocortisone, budesonide, fluticasone, cortisone acetate, and beclometasone including pharmaceutically acceptable esters, salts and complexes thereof. An especially suitable example is hydrocortisone or hydrocortisone 21-succinate or a pharmaceutically acceptable salt thereof.

With respect to the second part, any of the above-mentioned glucocorticoids may be employed. In a specific embodiment hydrocortisone is preferred.

### Pharmaceutical compositions

The present invention provides such glucocorticoid-containing pharmaceutical compositions and kits that are designed to release a first part of the glucocorticoid relatively fast in order to enable a fast on-set of action and to release a second part of the glucocorticoid in an extended manner in order to obtain a prolonged and sustained effect of the glucocorticoid. Preferably, the compositions and kits are designed for once daily administration. The glucocorticoid in the first part may be enhanced released (i.e. faster than normal) or immediate released.

Accordingly, a pharmaceutical composition comprises one or more glucocorticoids, wherein a first part of one or more glucocorticoids is substantially immediately released and a second part of one or more glucocorticoids is released over an extended period of time of at least about 8 hours.

Due to different potencies of the glucocorticoids, the term "hydrocortisone equivalents" dose exposure has been introduced.

The term "hydrocortisone equivalents" is used herein to define the amount in mg of a specific glucocorticoid that corresponds to 1 mg of hydrocortisone for the purpose of systemic glucocorticoid therapy as generally understood by medical practitioners. The term is based on the fact that the individual glucocorticoids have different potencies and in order to achieve a desired therapeutic effect different doses of the individual glucocorticoids are required. Equivalent doses of the glucocorticoids can be calculated based on the following table.

| Glucocorticoid | Equivalent amount (mg) | Hydrocortisone equivalent (1 mg of the glucocorticoid corresponds to the listed amount in mg of hydrocortisone) |
|---|---|---|
| Cortisone acetate | 25 | 0.8 |
| Hydrocortisone | 20 | 1 |
| Prednisolone | 5 | 4 |
| Prednisone | 5 | 4 |
| Methylprednisolone | 4 | 5 |
| Triamcinolone | 4 | 5 |
| Paramethasone | 2 | 10 |
| Betamethasone | 0.75 | 26.66 |
| Dexamethasone | 0.75 | 26.66 |
| Fludrocortisone | 0.05 | 400 |

Accordingly, if the first part of the composition contains 1.5 mg betamethasone (corresponding to 40 mg hydrocortisone) and the second part of the composition contains 40 mg hydrocortisone, the total amount of hydrocortisone equivalents in the composition corresponds to 80 mg hydrocortisone. Accordingly, the first part contains 50% of the total hydrocortisone equivalents of the composition. Assuming that the total amount of the glucocorticoid in the first part is released within 1 hour in the above-mentioned dissolution test, the requirement with respect to release of the glucocorticoid from the first part within the first 45 min is that at least 25% of the total hydrocortisone equivalents are released.

Thus in the compositions according to the invention the ratio of the glucocorticoids in the first part and the second part may be e.g. about 1:1, such as e.g. about 0.95:1,such as e.g. about 0.90:1, such as about 0.85:1, such as e.g. about 0.85:1, such as e.g. about 0.80:1, such as e.g. about 0.75:1, such as e.g. about 0.70:1, such as e.g. about 0.65:1, such as e.g. about 0.60:1, such as e.g. about 0.55:1, such as e.g. about 0.50:1, such as e.g. about 0.45:1, such as e.g. about 0.40:1, such as e.g. about 0.45:1, such as e.g. about 0.40:1, such as e.g. about 0.35:1, such as e.g. about 0.33:1, such as e.g. about 0.30:1, such as e.g. about 0.25:1, such as e.g. about 0.20:1, such as e.g. about 0.15:1, such as e.g. about 0.10:1, such as e.g. about 0.05:1 or such as e.g. about 0.01:1.

The amount of the one or more glucocorticoids of the first part, expressed as hydrocortisone equivalents, may be in a range of from about 15 to about 50%, notably from about 15% to about 35%, of the total hydrocortisone equivalents in the composition. This amount can be determined as the amount released 1 hour after start of testing of the composition in an in vitro dissolution test according to USP employing USP Dissolution Apparatus No. 2 (paddle), 50 rpm and simulated intestinal fluid without enzymes as dissolution medium and a temperature of 37 °C.

Normally, at least about 50% of the hydrocortisone equivalents of the first part are released within the first 45 min of the dissolution test.

A pharmaceutical composition according to the invention is suitably designed as a single composition intended for oral administration once daily. Such a composition is convenient for the patient to take and is therefore a preferred aspect. However, within the scope of the present invention a composition of the invention may also be a dual composition, i.e. including two different pharmaceutical forms, e.g. an extended release tablet to be ingested together with an immediate release oral pharmaceutical formulation of a glucocorticoid (or other suitable combinations). Such dual compositions are normally provided in a single package such as a kit. Accordingly, a kit may comprise
i) a first component comprising one or more glucocorticoids, the first component being designed for substantially immediately release of the one or more glucocorticoids,
ii) a second component comprising one or more glucocorticoids, the second component being designed for extended release of the one or more glucocorticoids,
wherein at least about 50% of the one or more glucocorticoids of the first component are released within the first 45 min of a dissolution test employing USP Dissolution Apparatus No. 2 (paddle), 50 rpm and simulated intestinal fluid without enzymes as dissolution medium.

The dissolution medium may be aqueous such as e.g. water optionally supplemented with a buffer, in a pH range of 3-8 such as about 4-7 such as about 5-6 or about 6. Furthermore, the temperature of the medium may be in range of e.g. 20-45°C, such as e.g. 25-40°C, such as e.g. 30-35°C or about 35-37°C.

The *in vitro* dissolution profiles of the glucocorticoid from drug formulations according to the invention is suitably followed over time in a standardized controlled *in vitro* environment. A United States Pharmacopoeia (USP) dissolution apparatus II (paddle) coupled to automatic sampling devices and software may be used for acquiring release profiles of the drug formulations in a neutral pH environment. The dissolution profile is suitably acquired at 37 °C, 50 rpm or 100 rpm of the paddles, in a total of 300 ml or 500 ml of water. Alternatively the water can be exchanged with phosphate buffer at pH=7.0. Sampling may be performed at even time intervals such as e.g. 0, 1, 3, 5, 7, 10, 15, 20, 30, 40 50 and 60 minutes following the insertion of a pharmaceutical composition according to the invention in the dissolution medium and may be followed up to 360 min or more after insertion of a pharmaceutical composition.

However, should a comparison on a composition according to the invention be made vis-à-vis a composition not belonging to the invention the comparison regarding the dissolution test should be made using a United States Pharmacopoeia (USP) dissolution apparatus II (paddle) coupled to automatic sampling devices and software was used for acquiring release profiles of the drug formulations in a neutral pH environment. The dissolution profile is acquired at 37 °C, 50 rpm of the paddles, in a total of 300 ml of water. Sampling is performed at 0, 1, 3, 5, 7, 10 and 15 minutes following the insertion of the pharmaceutical compositions. The release profile can be followed up to 360 minutes or more in even intervals.

In the present context the term "extended release" is intended to include all types of release which differ from the release obtained from plain tablets and that provide a release during 8 hours or more, which is a longer period of time than that obtained from plain tablets. Thus, the term includes so-called "controlled release", "dual release", "modified release", "sustained release", "sustained action" "pulsed release", "prolonged release", "slow release", "chrono-optimized release", continuous release, "time release", timed release" as well as the term "pH dependant release".

In the present context the term "immediate release" or "instant release" is intended to mean that the active substance (in this case the one or more glucocorticoids) begins to be released from the pharmaceutical composition immediately after being swallowed. In an *in vitro* testing this term is intended to mean that the active compound (e.g. the one or more glucocorticoids) that may be a part of a combined pharmaceutical composition (such as e.g. a combination of an immediate release part and an extended release part) is released from the composition according to the table below:

| | |
|---|---|
| time after start of the dissolution test | % hydrocortisone equivalents released (based on the content in the first part) |
| within 45 min | at least about 50% such as, e.g., at least about 60%, preferably at least about 70%, at least about 80% or at least about 90% |
| preferably within 30 min | at least about 50% such as, e.g., at least about 60%, preferably at least about 70%, at least about 80% or at least about 90% |
| within 20 min | at least about 50% such as, e.g., at least about 60%, at least about 70%, at least about 80% or at least about 90% |
| within 15 min | at least about 50% |

The *in vitro* dissolution profiles of the glucocorticoid from drug formulations (immediate and/or extended release) according to the invention is suitably followed over time in a standardized controlled *in vitro* environment. A United States Pharmacopoeia (USP) dissolution apparatus II (paddle) coupled to automatic sampling devices and software may be used for acquiring release profiles of the drug formulations in a neutral pH environment. The dissolution profile is suitably acquired at 37 °C, 50 rpm or 100 rpm of the paddles, in a total of 300 ml or 500 ml of water. Alternatively the water can be exchanged with phosphate buffer at pH=7.0. Sampling may be performed at even time intervals such as e.g. 0, 1, 3, 5, 7, 10, 15, 20, 30, 40 50 and 60 minutes following the insertion of a pharmaceutical composition according to the invention in the dissolution medium and may be followed up to 360 min or more after insertion of a pharmaceutical composition.

However, should a comparison on a composition according to the invention be made vis-à-vis a composition not belonging to the invention the comparison regarding the dissolution test should be made using a United States Pharmacopoeia (USP) dissolution apparatus II (paddle) coupled to automatic sampling devices and software was used for acquiring release profiles of the drug formulations in a neutral pH environment. The dissolution profile is acquired at 37 °C, 50 rpm of the paddles, in a total of 300 ml of water. Sampling is performed at 0, 1, 3, 5, 7, 10 and 15 minutes following the insertion of the pharmaceutical compositions. The release profile can be followed up to 360 minutes or more in even intervals.

By using one or more glucocorticoids for immediate release and one or more glucocorticoids for extended release in specific ratios, it has been possible to mimic the circadian rhythm of cortisol after administration. Moreover, it may be envisaged that it is possible to lower the daily dosage range required to obtain a suitable therapeutic effect taking into consideration the general release profile differences in individual patients their sensitivity to the drug, and their body weights. Thus, for an average adult person, whose endogenous cortisol excretion is at a very low or zero level, the total daily dose of hydrocortisone in the range of 15-30 mg or equivalent doses of other glucocorticoids can be administered once a day in order to essentially mimic the endogenous release profile. In the present context, the term "essentially mimic" is intended to denote that the plasma profile obtained in a time period corresponding to from about 0.5-1 to about 6.5-7 hours after administration of the composition or a kit according to the invention substantially imitates or resembles the shape of the plasma profile of cortisol of a healthy subject in the morning from 6am to noon. In the case that the first and the second parts (or components in the case of a kit) of the one or more glucocorticoids are taken sequentially, the time period runs from administration of the first part.

The pharmaceutical composition or kit of the invention should provide intestinal drug absorption for about 12-18 hours after dosing.

In the following is given a detailed description of the invention relating to pharmaceutical composition. However, all details and particulars disclosed under this aspect of the invention apply *mutatis mutandis* to the other aspects of the invention. Especially, it should be noted that disclosure relating to the first and/or the second parts of a composition according to the invention also applies for a first and second component of a kit according to the invention.

Consequently, a composition according to the invention may thus comprise (wherein the percentages are given as % of total weight of all ingredients used in the manufacture of the composition). It is also to be understood that the solvent used on the process of manufacturing the composition may fully or partly evaporate:

| Glucocorticoid | about 1.5% to about 6.5% |
|---|---|
| Polymer, Binder | about 15-25%, such as about 20% |
| Filler | about 30-40%, such as about 35% or about 37% |
| Glidant | about 0.1-0.5%, such as about 0.3% |
| Lubricant | about 0.1-0.5%, such as about 0.3% |
| Film coating system | about 1-5%, such as about 3.5% or about 4.5% |
| Solvent | about 25-40%, such as about 30% or about 35% |

Thus a composition according to the invention may e.g. have the following make-up:

| Glucocorticoid | about 1.6% |
|---|---|
| Polymer, Binder | about 22% |
| Filler | about 38% |
| Glidant | about 0.3% |
| Lubricant | about 0.3% |
| Film coating system | about 4.4% |
| Solvent | about 33% |

or may e.g. have the following make-up:

| Glucocorticoid | about 6.2% |
|---|---|
| Polymer, Binder | about 20% |
| Filler | about 36% |
| Glidant | about 0.3% |
| Lubricant | about 0.3% |
| Film coating system | about 3.4% |
| Solvent | about 33% |

or e.g.:

| Glucocorticoid | about 4.5% |
|---|---|
| Polymer, Binder | about 22% |
| Filler | about 36% |
| Glidant | about 0.3% |
| Lubricant | about 0.3% |
| Film coating system | about 3.9% |
| Solvent | about 33% |

Examples of suitable fillers, diluents and/or binders include lactose (e.g. spray-dried lactose, α-lactose, β-lactose, Tabletose®, various grades of Pharmatose®, Microtose® or Fast-Floc®), microcrystalline cellulose (various grades of Avicel®, Elcema®, Vivacel®, Ming Tai® or Solka-Floc®), hydroxypropylcellulose, L-hydroxypropylcellulose (low substituted), hydroxypropyl methylcellulose (HPMC) (e.g. Methocel E, F and K, Metolose SH of Shin-Etsu, Ltd, such as, e.g. the 4,000 cps grades of Methocel E and Metolose 60 SH, the 4,000 cps grades of Methocel F and Metolose 65 SH, the 4,000, 15,000 and 100,000 cps grades of Methocel K; and the 4,000, 15,000, 39,000 and 100,000 grades of Metolose 90 SH), methylcellulose polymers (such as, e.g., Methocel A, Methocel A4C, Methocel A15C, Methocel A4M), hydroxyethylcellulose, sodium carboxymethylcellulose, carboxymethylene, carboxymethylhydroxyethylcellulose and other cellulose derivatives, sucrose, agarose, sorbitol, mannitol, dextrins, maltodextrins, starches or modified starches (including potato starch, maize starch and rice starch), calcium phosphate (e.g. basic calcium phosphate, calcium hydrogen phosphate, dicalcium phosphate hydrate), calcium sulfate, calcium carbonate, sodium alginate, collagen etc.

Specific examples of diluents are e.g. calcium carbonate, dibasic calcium phosphate, tribasic calcium phosphate, calcium sulfate, microcrystalline cellulose, powdered cellulose, dextrans, dextrin, dextrose, fructose, kaolin, lactose, mannitol, sorbitol, starch, pregelatinized starch, sucrose, sugar etc.

Specific examples of disintegrants are e.g. alginic acid or alginates, microcrystalline cellulose, hydroxypropyl cellulose and other cellulose derivatives, croscarmellose sodium, crospovidone, polacrillin potassium, sodium starch glycolate, starch, pregelatinized starch, carboxymethyl starch (e.g. Primogel® and Explotab®) etc.

Specific examples of binders are e.g. acacia, alginic acid, agar, calcium carrageenan, sodium carboxymethylcellulose, microcrystalline cellulose, dextrin, ethylcellulose, gelatin, liquid glucose, guar gum, hydroxypropyl methylcellulose, methylcellulose, pectin, PEG, povidone, pregelatinized starch etc.

Glidants and lubricants may also be included in the composition. Examples include stearic acid, magnesium stearate, calcium stearate or other metallic stearate, talc, waxes and glycerides, light mineral oil, PEG, glyceryl behenate, colloidal silica, hydrogenated vegetable oils, corn starch, sodium stearyl fumarate, polyethylene glycols, alkyl sulfates, sodium benzoate, sodium acetate etc.

Other excipients which may be included in a composition or solid dosage form of the invention are e.g. flavouring agents, colouring agents, taste-masking agents, pH-adjusting agents, buffering agents, preservatives, stabilizing agents, anti-oxidants, wetting agents, humidity-adjusting agents, surface-active agents, suspending agents, absorption enhancing agents, agents for modified release etc.

The composition or kit components may also be coated with a film coating, an enteric coating, a modified release coating, a protective coating, an anti-adhesive coating etc.

A composition (or part thereof) may also preferably be coated in order to obtain suitable properties e.g. with respect to extended release of the one or more glucocorticoids. The coating may also be applied as a readily soluble film containing the one or more glucocorticoids for immediate release. The coating may also be applied in order to mask any unsuitable taste of the one or more glucocorticoids. The coating may be applied on single unit dosage forms (e.g. tablets, capsules) or it may be applied on a polydepot dosage form or on its individual units.

Suitable coating materials are e.g. methylcellulose, hydroxypropylmethylcellulose, hydroxypropylcellulose, acrylic polymers, ethylcellulose, cellulose acetate phthalate, polyvinyl acetate phthalate, hydroxypropyl methylcellulose phthalate, polyvinylalcohol, sodium carboxymethylcellulose, cellulose acetate, cellulose acetate phthalate, gelatin, methacrylic acid copolymer, polyethylene glycol (Macrogol), shellac, sucrose, titanium dioxide, carnauba wax, microcrystalline wax, glyceryl monostearate, talc, zein, colorants or pigments or any combinations thereof..

Plasticizers and other ingredients may be added in the coating material. The same or different active substance may also be added in the coating material.

It is to be clearly understood that the excipients mentioned herein may be present in any combinations in a final pharmaceutical formulation according to the invention, i.e may contain any combinations of binders with e.g. lubricants/glidants etc.

The compositions as mentioned above may have a ratio between the first immediate release part (which may e.g. be the coating of a tablet)and the second extended release part (which may be the core of a tablet) in ranges of 20-30% such as e.g. about 25% of the glucocorticoid for the coating (first part being immediate release) of the total amount of glucocorticoid and 70-80% such as about 75% of the core (second part being extended release) of the total amount of glucocorticoid. The film-former used in a coating composition may comprise polyvinyl alcohol, macrogol, talc and titanium oxide.

The solvents used in the manufacturing process of the compositions according to present invention may be any aqueous solvent such as water optionally supplemented an alcohol such as e.g. methanol or ethanol. The solvent may also be an organic solvent such as ethanol, methanol, isopropylalcohol, dichloromethane or the likes.

A concrete non-limiting example of a composition according to the invention may be compositions formulated as coated tablets for oral administration comprising either 5 mg or 20 mg of hydrocortisone. The composition may have a core structure that is coated and the core may be formulated to release the active substance in an extended release fashion as discussed above. The coating may be formulated to release the active substance in an immediate fashion as discussed above. Furthermore a composition according to the invention may have the following constituents:

| **Ingredient** | **Quantity (5 mg tablet), mg/unit** | **Quantity (20 mg tablet), mg/unit** | **Standard** |
|---|---|---|---|
| Hydrocortisone | 5.0 | 20.0 | Ph.Eur. |
| Hypromellose K 100 cP (Methocel K 100) | 47.05 | 41.2 | Ph.Eur. |
| Hypromellose K 4000 cP (Methocel K4M) | 20.0 | 24.6 | Ph.Eur. |
| Cellulose, microcrystalline (Avicel PH-102) | 100.8 | 100.8 | Ph.Eur. |
| Starch, pregelatinized (Starch 1500) | 16.4 | 16.4 | Ph.Eur. |
| Silica colloidal anhydrous (Aerosil 200) | 1.0 | 1.0 | Ph.Eur. |
| Magnesium stearate | 1.0 | 1.0 | Ph.Eur. |
| Opadry II | about 13.75 | about 11.0 | Colorcon |
| Water, purified* | about 102 | about 107 | Ph.Eur. |

| | | | |
|---|---|---|---|
| ** Evaporates during the manufacturing process* | | | |

The formulations may be of type swelling matrices based on hypromellose and a direct compression technique can be selected in the manufacturing process of the tablets.

The core tablets may thus comprise e.g. hydrocortisone, hypromellose, microcrystalline cellulose, starch pregelatinized, silica and magnesium stearate that are mixed and compressed to tablets of high resistance to crushing. The tablets may be circular convex tablets.

The core tablets are preferably coated with e.g. hydrocortisone and a film former (e.g. Opadry® and water to obtain hydrocortisone for immediate release. The film-former used in the coating composition comprise e.g. polyvinyl alcohol, macrogol (polyethylene glycol), talc and titanium oxide and optionally a colorant/pigment. The compositions described above may in the case of a 5 mg tablet have an amount of 1.25 mg of the active pharmaceutical ingredient (such as e.g. hydrocortisone) in the coating and 3.75 mg of the active pharmaceutical ingredient (such as e.g. hydrocortisone) in the core. Furthermore, in the 20 mg tablet the coating may have an amount of 5 mg of the active pharmaceutical ingredient (such as e.g. hydrocortisone) and an amount of 15 mg of the active pharmaceutical ingredient (such as e.g. hydrocortisone) in the core. Furthermore, the amount of the one or more glucocorticoids of the first part, expressed as hydrocortisone equivalents, may be in a range of from about 15 to about 50%, notably from about 15% to about 35% or from about 20% to about 40%, such as e.g. from about 25% to about 35%, of the total hydrocortisone equivalents in the composition.

The function of each of the excipients is provided in the table, below.

### Function of excipients

| Ingredient | Function |
|---|---|
| Hypromellose K 100 cP (Methocel K 100) | Polymer, Binder |
| Hypromellose K 4000 cP (Methocel K4M) | Polymer, Binder |
| Cellulose, microcrystalline (Avicel PH-102) | Filler |
| Starch, pregelatinized (Starch 1500) | Filler |
| Silica colloidal anhydrous (Aerosil 200) | Glidant |
| Magnesium stearate | Lubricant |
| Opadry II | Film coating system |
| Water, purified | Solvent |

Thus a composition according to the invention may comprise any of the above listed pharmaceutical excipients in any combination together with one or more glucocorticoids.

Further pharmaceutical compositions and methods of producing these as well as release patterns/profiles can be found in PCT/EP2005/004400 and published as WO/2005/102271 which is incorporated herein by reference.

Thus the invention relates to compositions for use in administrations where the administration may be any combination of different unit dosages such as e.g. a combination of any number of 5 mg dosages and any number 20 mg dosages. Thus if the total daily dose of e.g. 25 mg of hydrocortisone (or hydrocortisone equivalent) is required, the subject in need thereof is administered one tablet comprising 20 mg of hydrocortisone (or hydrocortisone equivalent) and a tablet comprising 5 mg of hydrocortisone (or hydrocortisone equivalent) either simultaneously or within a time interval as specified above. Depending on the required dosage intervals the total daily dose of e.g. 25 mg can be administered as 5 different 5 mg tablets comprising hydrocortisone (or hydrocortisone equivalent).

The invention also relates to compositions where the total daily dose of glucocorticoids (stated as hydrocortisone equivalents) that is administered to a subject in need thereof is e.g. about 1 mg or about 5 mg or about 10 mg or about 15 mg or about 20 mg or about 25 mg or about 30 mg or about 40 mg or about 50 mg or about 60 mg or about 70 mg or about 80 mg or about 90 mg or about 100 mg or about 150 mg or about 200 mg depending upon the clinical requirement.

The administration of the composition according to the invention may be any solid dosage form for oral administration or it may be in any form suitable for parenteral administration. The composition may be formulated e.g. as a tablet, capsule, pill, or a sachet. Parenteral administration may be e.g. intravenous, subcutaneous, intraarterial or intramuscular.

### Figures

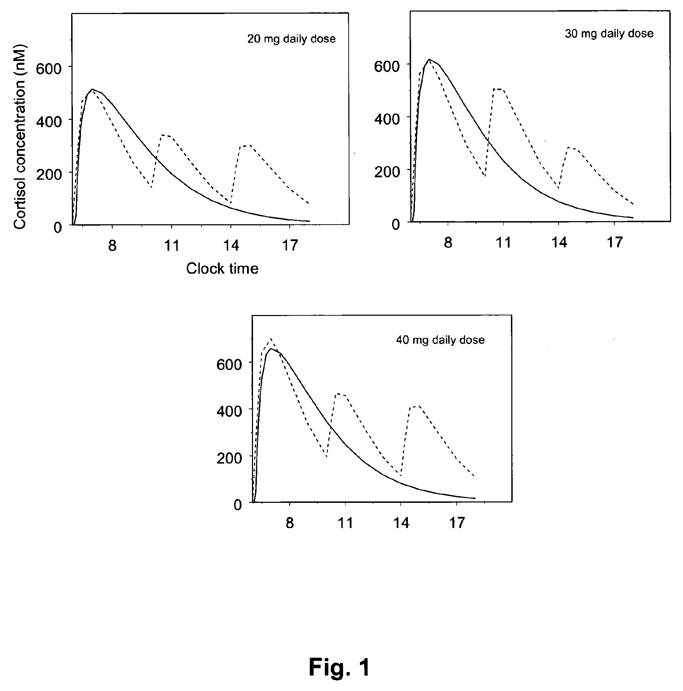

**Figure 1****.** Illustrates the cortisol concentration (nM) vs. clock time in a typical patient (80 kg) after 20, 30 or 40 mg total daily dose of hydrocortisone in a composition according to the invention administered once daily (solid line) or hydrocortisone tablet (Hydrocortone, Merck Sharp & Dome) three times daily (dotted line, 20 mg; 10+5+5mg, 30 mg; 15+10+5mg, 40 mg; 20+10+10 mg)

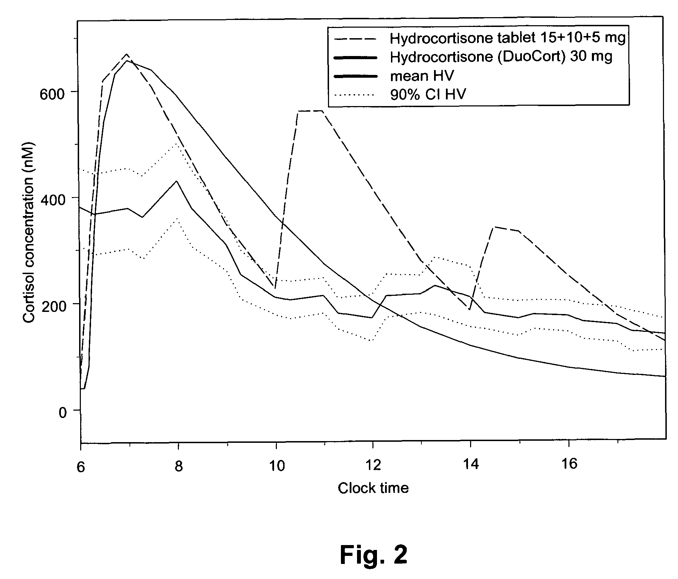

**Figure 2****.** Illustrates the cortisol concentration (nM) vs. clock time in a typical patient (80 kg) after 30mg of hydrocortisone in a composition according to the invention administered once daily or hydrocortisone tablet (Hydrocortone, Merck Sharp & Dome) administered three times daily (30 mg; 15+10+5mg)

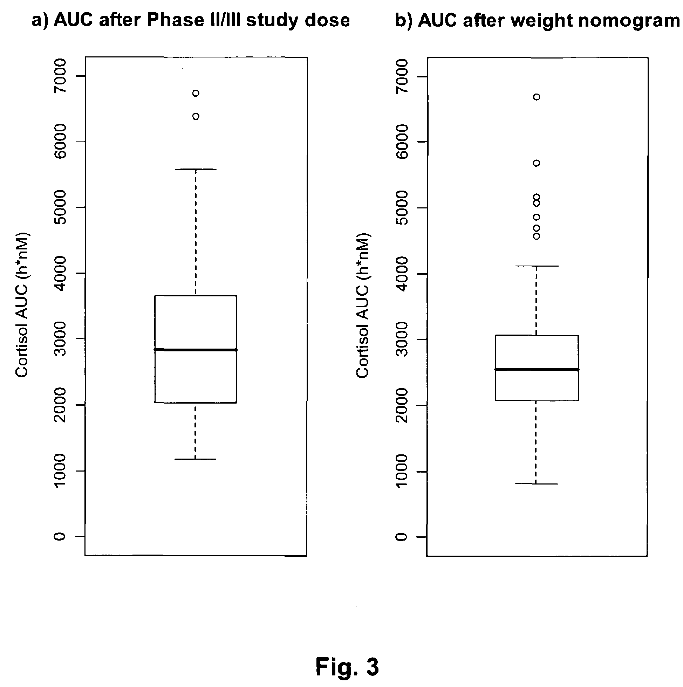

**Figure 3****.** Illustrates box-plots of predicted Hydrocortisone in a composition according to the invention with total AUC based on the final population pharmakokinetic model for a) individual doses given in a Phase II/III study (best clinical practice) and b) doses given by the weight nomogram according to the invention. The box shows the interquartile range and median.

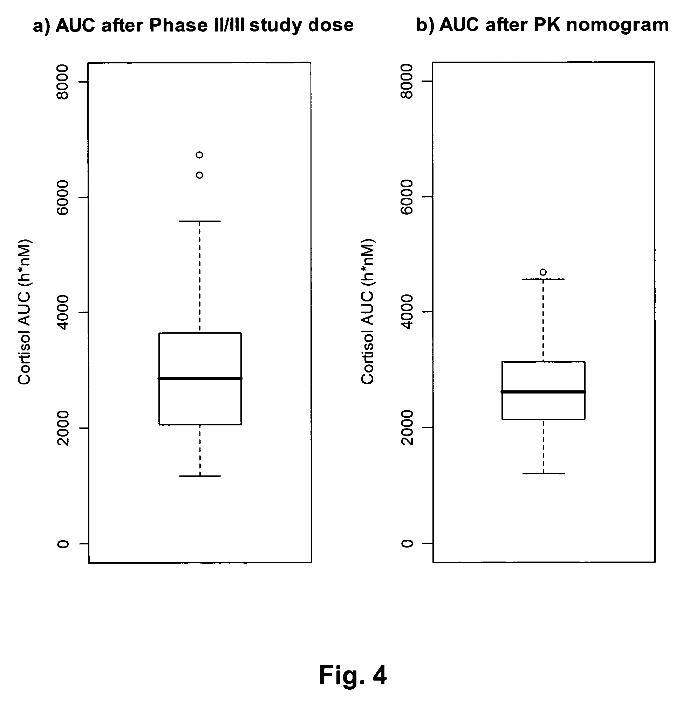

**Figure 4****.** Illustrates box-plots of predicted Hydrocortisone in a composition according to the invention with total AUC based on the final population pharmakokinetic model for a) individual doses given in a Phase II/III study (best clinical practice) and b) doses given by the pharmakokinetic nomogram according to the invention. The box shows the interquartile range and median.

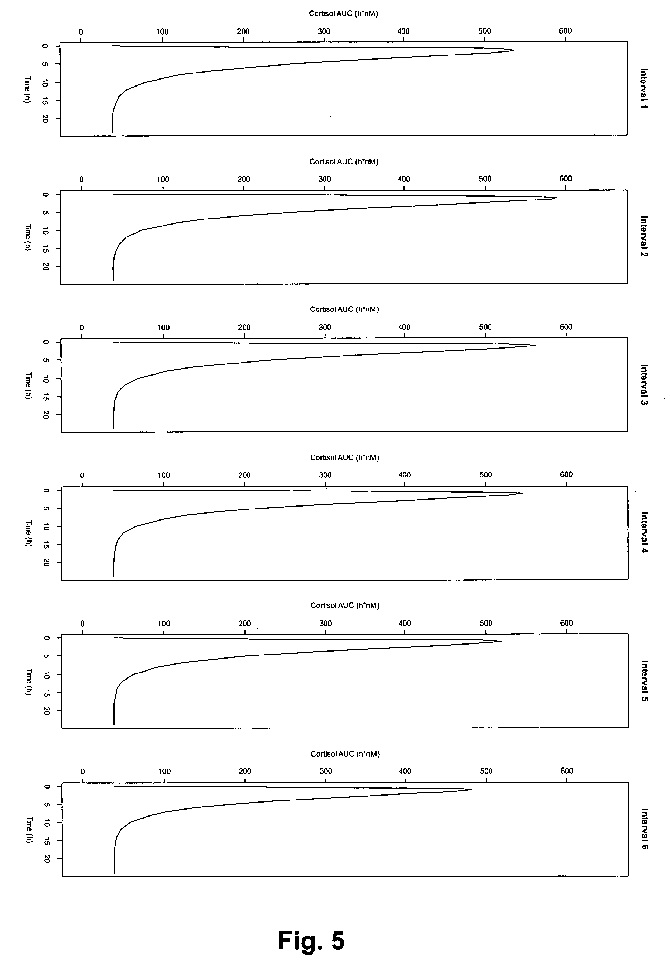

**Figure 5****.** Average cortisol concentrations versus time after PK nomogram doses and C₀ₕ=40 nM (typical value) and lower limit in the different C_{cortisol} intervals (Intervals 1-6).

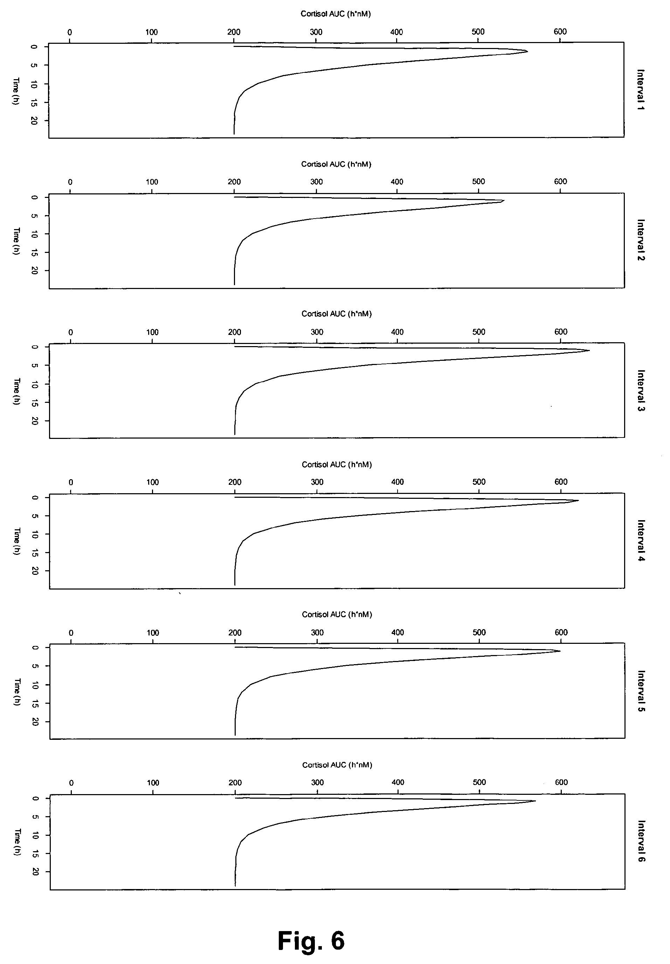

**Figure 6****.** Average cortisol concentrations versus time after PK nomogram doses and 150<C₀ₕ≤250 nM and lower limit in the different C_{cortisol} intervals (Intervals 1-6).

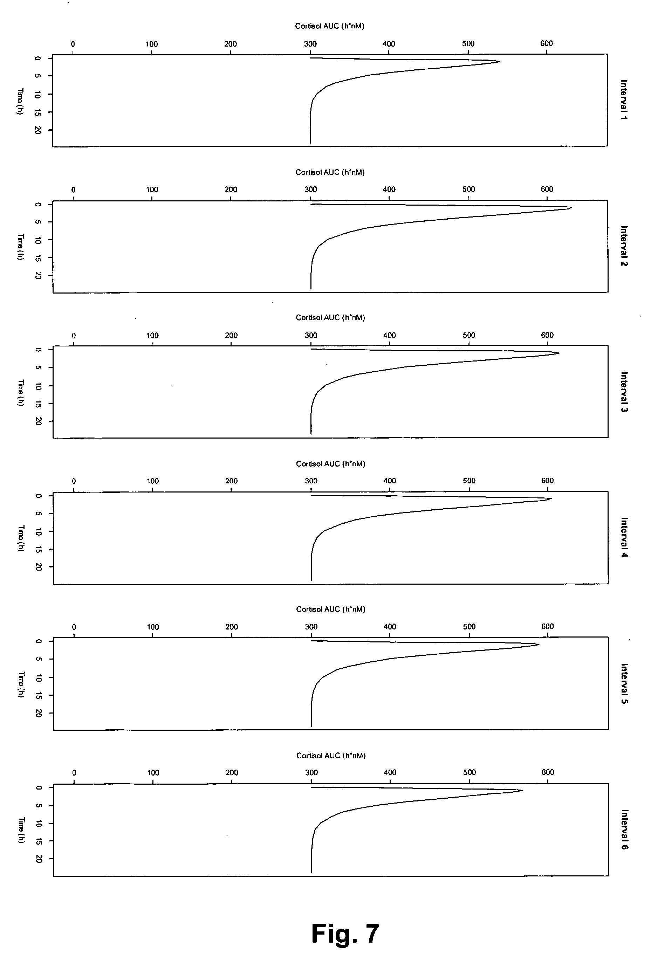

**Figure 7****.** Average cortisol concentrations versus time after PK nomogram doses and 250<C₀ₕ≤350 nM and lower limit in the different C_{cortisol} intervals (Intervals 1-6).
**Figure 8****.** Individual simulated cortisol concentrations vs. time after the first dose in the Al patients in clinical study after hydrocortisone in a composition according to the invention is given as a) 30 mg + 30 mg 6 hrs post first dose, b) 30 mg + 30 mg 8 hrs post first dose, c) 30 mg + 30 mg 10 hrs post first dose d) 30 mg + 30 mg 12 hrs post first dose, e) 30 mg + 30 mg 8 hrs post first dose + 30 mg 16 hrs post first dose, f) 2 x 30 mg and g) 30 mg + 30 mg 12 hrs post first dose for two consecutive days

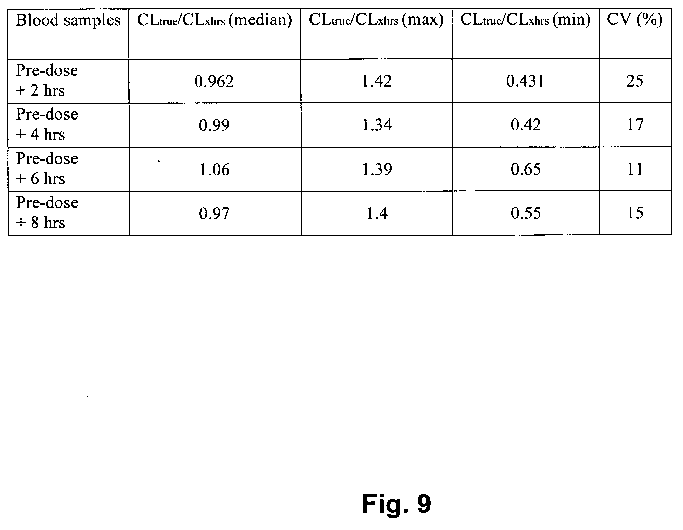

**Figure 9****.** Illustrates the results from the evaluation of optimal sampling time points fro PK nomograms. As seen from the table the optimal sampling time was found to be 6 hours after taking the first blood sample immediately before administering the composition according to the invention.

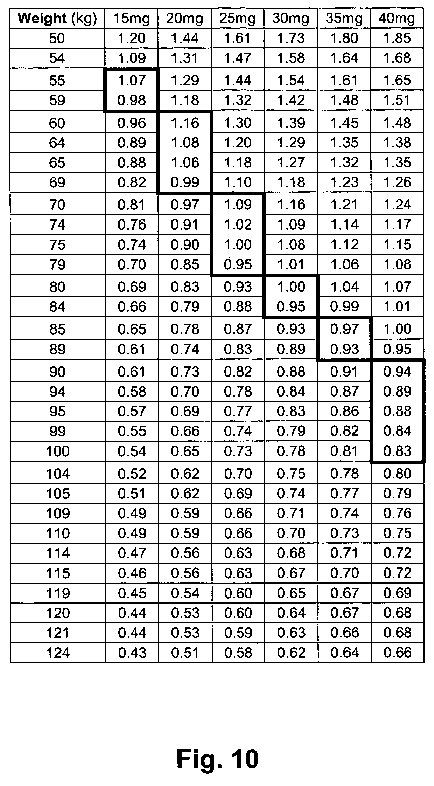

**Figure 10****.** Illustrates the AUC ratios (of exposure at each weight level/target exposure) for different doses. A ratio of 1 indicates that the dose will give the target exposure. A ratio<1 indicates lower exposure compared to target. A ratio>1 indicates a higher exposure than target. Boxed cells indicate the selected dose levels for each weight interval.

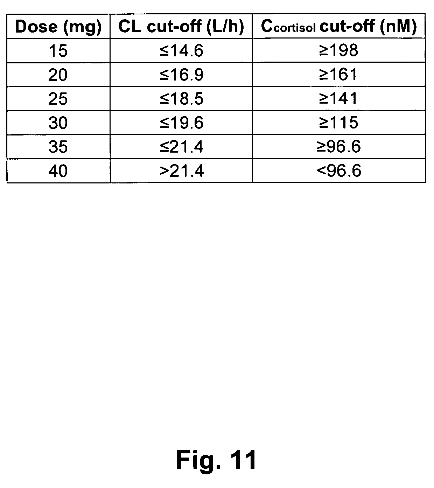

**Figure 11****.** Illustrates the CL cut-off values and corresponding Cₒᵣₜᵢₛₒₗ cut-off values for the final PK nomogram.

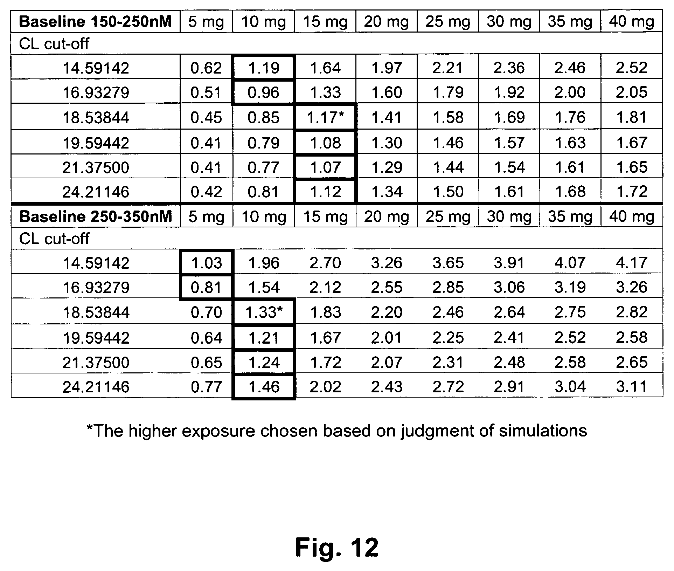

**Figure 12****.** Illustrates AUC ratio (of exposure at each CL level/target exposure) for different doses. An AUC ratio of 1 indicates that the dose will give the target exposure.
A ratio<1 indicates lower exposure compared to target. A ratio>1 indicates a higher exposure than target. Boxed cells indicate the selected dose levels for each CL cut-off.

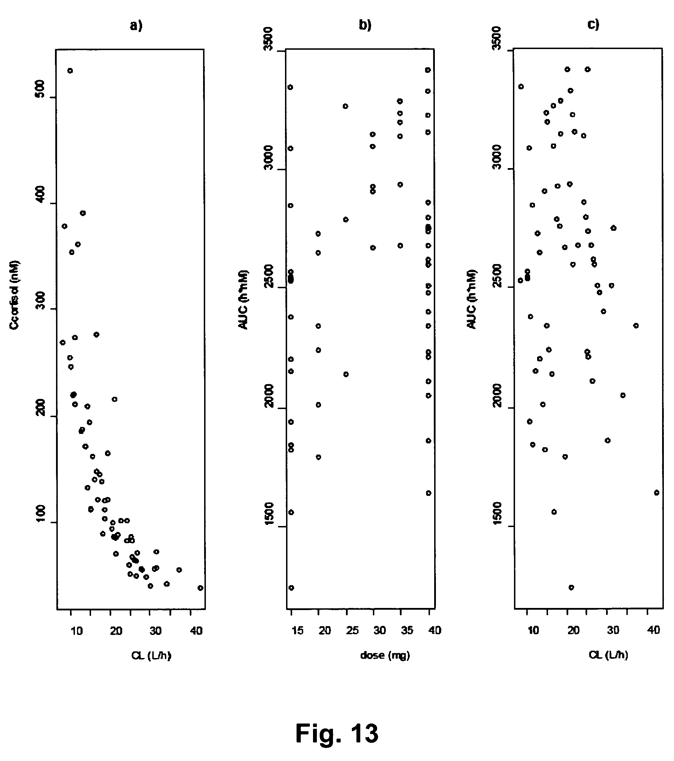

**Figure 13** Illustrates the evaluation of final PK nomogram using final and adjusted CLcut-off values. a) Predicted individual Ccortisol (C6h-C₀ₕ) after 20 mg of the composition according to the invention versus individual CL in patients (n=62) at first occasion. b) Predicted individual cortisol AUC versus predicted dose based on PK nomogram and c) Predicted individual cortisol AUC after PK nomogram dose versus predicted CL based on the PK model according to the invention.

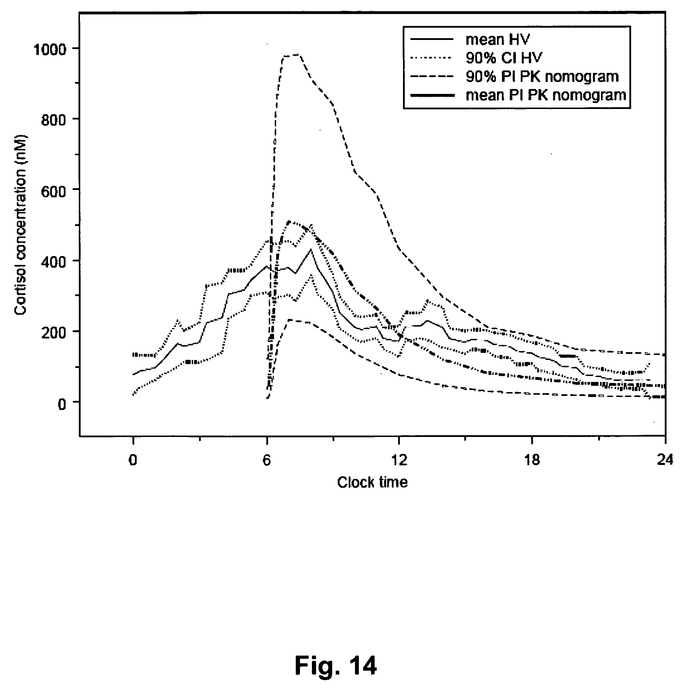

**Figure 14** Illustrates the simulated mean and 90% prediction interval (PI) after doses based on PK nomogram according to the invention. Observed mean cortisol concentration versus clock time and 90% confidence interval (CI) in healthy volunteers (HV).

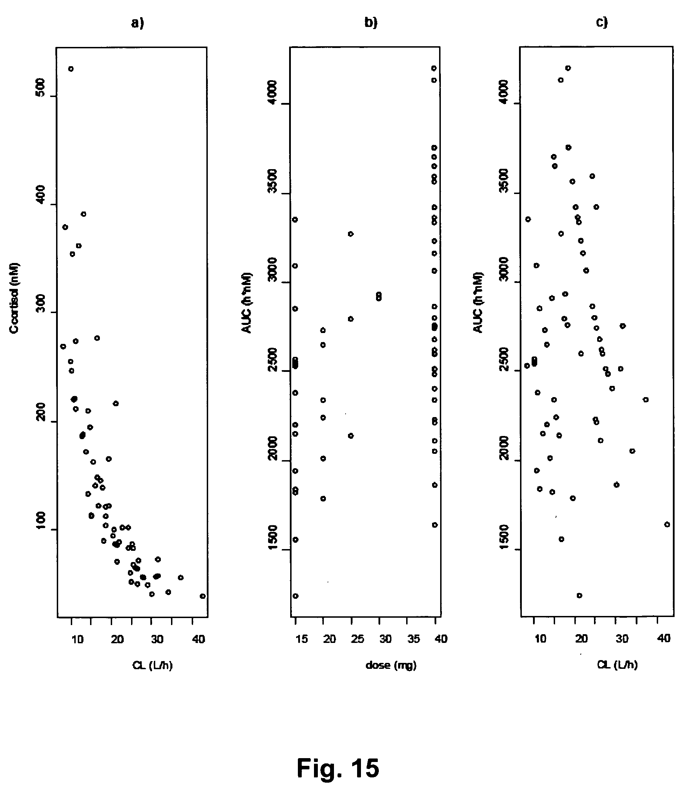

**Figure 15** Illustrates the evaluation of PK nomogram using CL cut-off values where AUC/AUCtarget was as close to unit as possible. a) Predicted individual C_{cortisol} (C₆ₕ-C₀ₕ) after 20 mg of the composition according to the invention versus individual CL in patients (n=62) at first occasion. b) Predicted individual cortisol AUC versus predicted dose based on PK nomogram and c) Predicted individual cortisol AUC after PK nomogram dose versus predicted CL based on the PK model

### Abbreviations:

Al abbreviates Adrenal Insufficient or Adrenal Insufficiency
o.d. abbreviates once daily
t.i.d. abbreviates three times daily
AUC abbreviates Area Under the Curve
PK abbreviates PharmacoKinetic
PD abbreviates PharmacoDynamic
CL/F abbreviates oral clearance
IOV abbreviates interoccasional variability
OFV abbreviates objective functional values
CV abbreviates coefficient of variation
CL abbreviates clearance

### Examples

### Reference Example 1

The hydrocortisone composition used herein in a 5 mg or 20 mg dose of hydrocortisone has the following constitution;

| **Ingredient** | **Quantity (5 mg tablet), mg/unit** | **Quantity (20 mg tablet), mg/unit** | **Standard** |
|---|---|---|---|
| Hydrocortisone | 5.0 | 20.0 | Ph.Eur. |
| Hypromellose K 100 cP (Methocel K 100) | 47.05 | 41.2 | Ph.Eur. |
| Hypromellose K 4000 cP (Methocel K4M) | 20.0 | 24.6 | Ph.Eur. |
| Cellulose, microcrystalline (Avicel PH-102) | 100.8 | 100.8 | Ph.Eur. |
| Starch, pregelatinized (Starch 1500) | 16.4 | 16.4 | Ph.Eur. |
| Silica colloidal anhydrous (Aerosil 200) | 1.0 | 1.0 | Ph.Eur. |
| Magnesium stearate | 1.0 | 1.0 | Ph.Eur. |
| Opadry II | about 13.75 | about 11.0 | Colorcon |
| Water, purified* | about 102 | about 107 | Ph.Eur. |

The compositions described above have in the case of a 5 mg tablet an amount of 1.25 mg of the active pharmaceutical ingredient (hydrocortisone) in the coating and 3.75 mg of the active pharmaceutical ingredient hydrocortisone) in the core. Furthermore, in the 20 mg tablet the coating has an amount of 5 mg of the active pharmaceutical ingredient (hydrocortisone) and an amount of 15 mg of the active pharmaceutical ingredient (hydrocortisone) in the core.

### Example 1. Population Pharmacokinetic Modelling (POPPK)

### Population PK modelling of Hydrocortisone in a composition according to Reference Example 1

Cortisol plasma/serum concentration versus time data was pooled from the two studies in order to provide a dose range of 5-60 mg Hydrocortisone (DuoCort) for PK model development. One dataset contained plasma cortisol concentration time data from a Phase I study in healthy volunteers and one Phase II/III dataset contained serum cortisol concentration versus time data in primary adrenal insufficient patients. Only data from the fasted condition and analysed with immunoassay method were used from the Phase I study. The PK from the Phase II/III study was studied in the fasted state.

The rational for combining both patient and healthy volunteer data was that early analysis of only patient data revealed that some patients would benefit from a dose lower than 20 mg, based on the identified relationship between oral clearance and body weight, between patient variability and clinical target criteria. In order to develop the weight and PK nomograms to include recommendations of a dose of 15 mg, a POPPK model including data below 20 mg had to be developed due to the nonlinearity decrease in bioavailability with increasing doses. Bridging between patient and healthy volunteers was facilitated due to the common dose of 20 mg in the fasted state.

The data included in the POPPK analysis of hydrocortisone composition according to reference example 1 was venous blood collected in 62 patients after oral o.d.(once daily) administration of the modified-release formulation Hydrocortisone (DuoCort) at a total of 116 occasions where serum concentrations versus time profiles were obtained. The hydrocortisone dose range in patients was 20-60 mg o.d.. Venous blood was collected for rich plasma concentration versus time profiles in healthy volunteers (13 subjects included in the POPPK analysis) after single oral administration of 5 mg and 20 mg Hydrocortisone composition according to reference example 1 at two different randomised occasions. The endogenous cortisol production in the healthy volunteers was prior to Hydrocortisone composition according to reference example 1 administration suppressed by administering betamethasone. The body weight range in the pooled dataset was 53-122 kg with a median weight of 76 kg. The median body weight in the healthy volunteer and patient populations were 68 and 80 kg, respectively.

All cortisol concentration versus time data were simultaneous modeled using a nonlinear mixed effects modeling approach in NONMEM (version 6.0. ICON Development Solutions) with the first-order conditional estimation method (ICON, Hanover, MD, USA; Beal et al. 1989). Different PK models were explored and potentially PK differences between patients and healthy volunteers were described. The model predicted typical cortisol exposure, defined as the total area under the cortisol concentration time curve (AUC_{(0-infinity}) in a typical patient after different doses of Hydrocortisone composition according to reference example 1, were compared to exposure in un-suppressed healthy volunteers (Vgontzas et al. 2001) as well as to predefined target profile criteria for the pharmaceutical development in order to define a clinical exposure target. Based on the derived clinical exposure target and the POPPK model, a body weight nomogram and a PK nomogram were developed to facilitate clinical *a priori* and *a posteriori* dose individualization. In the dose range 15-40 mg with 5 mg increments, doses were derived where the exposure would be as close as possible to the clinical target exposure based on typical model parameters (fixed effects) and different body weight ranges. The weight nomogram was validated for each patient in the clinical study by simulating the exposure after a hydrocortisone (composition according to reference example 1) dose given by the weight nomogram and comparing this to the model-predicted exposure after the dose given in the study.

The PK nomogram was constructed by first investigating, using simulations from the POPPK model, at what time (after dose), a blood sample would contain most information about oral clearance, the only determinate for exposure apart from dose. Thereafter, simulations were used to predict the interval for 6 hours post-dose serum concentrations for each 5 mg increment dose interval in the dose range 15-40 mg. The PK nomogram was validated for each patient in the clinical study by simulating the exposure after a hydrocortisone dose (composition according to reference example 1) given by the PK nomogram and comparing this to the model-predicted exposure after the dose given in the study. The final PK model was also used to simulate different dosing regimens for intercurrent illness.

### Population PK modelling of hydrocortisone tablet

Cortisol concentration versus time data after different doses of hydrocortisone tablet three times daily (t.i.d.) (Hydrocortone^{®}, MSD) in primary Al patients was included in the analysis. All cortisol concentration versus time data from patients were simultaneous modeled using a nonlinear mixed effects modeling approach in NONMEM (version6.0) with the first-order conditional estimation method (ICON, Hanover, MD, USA; Beal et al. 1989). The data included in the POPPK analysis was venous blood collected in 63 patients after oral t.i.d. administration of hydrocortisone tablets at a total of 364 dosing occasions where serum hydrocortisone concentrations versus time profiles were obtained. The dose range in patients was 20-40 mg. The total daily dose was dived as follows: 20 mg; 10+5+5mg, 25 mg; 15+5+5 mg, 30 mg; 15+10+5mg, 40 mg; 20+10+10 mg and given at 8:00, 12:00 and 16:00 o'clock. The body weight range was 54-122 kg with a median weight of 80 kg.

### Population PK model selection and evaluation

Model selection was based on objective function values (OFV), which is minus twice the loglikelihood of the data, as well as goodness-of-fit plots, standard errors, visual predictive checks and scientific plausibility. A drop in the OFV of 3.84 between two nested models corresponds approximately to p< 0.05 which was regarded as statistically significant. Visual predictive checks were done in order to evaluate the predictive performance of the models.

### Results of Population PK modelling of Hydrocortisone in a composition according to reference example 1

Hydrocortisone (composition according to reference example 1), PK in patients and suppressed healthy volunteers was best described by a two-compartmental model with first order absorption with a lag-time. In the dose range 5-60 mg, the cortisol bioavailability after Hydrocortisone (composition according to reference example 1) administration decreased non-linearly with increasing doses resulting in the lack of dose-proportional increase in exposure with dose. This is most likely due to reduced dissolution rate *in vivo* at higher oral doses. The bioavailability was predicted to be 36% lower after 40 mg compared to 20 mg Hydrocortisone (composition according to reference example 1). A relationship between oral clearance, the individual predictor for exposure, and body weight was found which formed the basis for the development of the weight nomogram. No significant relation between Hydrocortisone (composition according to reference example 1) PK and age, renal function [(Modified Diet in Renal Disease (MDRD)] or gender was supported in the POPPK analysis.

Differences in cortisol PK after Hydrocortisone (composition according to reference example 1) administration were estimated between suppressed healthy volunteers and patients using the population PK modelling approach of PK data from the Phase I study Phase II/III study. Oral clearance and volume of distribution of the central compartment were estimated to be 17% and 30% lower, respectively, in suppressed healthy volunteers as compared to patients. Hydrocortisone is a low extraction drug (Lennernäs et al 2008) and the fraction unbound is therefore directly correlated to oral clearance and volume of distribution. Long-term treatment with glucocorticoids may reduce the serum concentration of corticosteroid-binding globulin (Axelrod 2006). The serum concentration of binding proteins is therefore likely to be higher in healthy volunteers resulting in a lower unbound fraction and thereby a lower oral clearance and volume of distribution.

IIV (Inter Individual Variability) in oral clearance was estimated to be 23% which is judged to be moderate to high IIV. This means that when all other predictors of variability in exposure have been taken into account (ie dose and body weight) there is still a fairly high variability between patients. This formed the basis for the development of the PK nomogram. Interoccasional variability (IOV) was estimated to be 20% in oral clearance and 76% in central volume of distribution. Rate of absorption was predicted to be faster after the 5 mg dose (0.63 h⁻¹) as compared to the other dose levels (0.44 h⁻¹). A likely explanation to the difference in absorption rate at low doses is the dissolution dependent absorption rate of this BCS class II drug (Amidon et al 1995; Lennernäs et al 2008).

In some healthy volunteers, the endogenous cortisol production was not fully suppressed resulting in pre-dose cortisol concentrations above the limit of quantification. Interestingly, some patients also had pre-dose cortisol concentrations indicating residual cortisol production. In order to account for pre-dose cortisol levels in both patients and suppressed healthy volunteers, the predicted cortisol concentration was modelled as a sum of exogenous cortisol due to Hydrocortisone (composition according to reference example 1) administration and endogenous cortisol production. The endogenous cortisol baseline level was on average estimated to be 40 and 19 nM in patients and healthy volunteers, respectively, with a large IIV (66%). The endogenous cortisol level approach assumes a constant baseline shift in hydrocortisone profiles during the entire 24 hour dosing interval. This is most likely valid for the patient data since no circadian rhythm is likely to remain. However, for the healthy volunteer data, the 24 hour post-dose cortisol concentration was in many individuals higher than the second last sample. Betamethasone was administered with 8 hours intervals. The effect of dexamethasone is sustained for at least 24 hours (Hong et al. 2007) and dexamethasone and betamethasone have similar glucocorticoid potency as compared with hydrocortisone (Lipai et at 1992). However, in order to describe all 24 hours post-dose samples in healthy volunteers, IOV was introduced in the endogenous cortisol levels by dividing a PK sampling occasion in healthy volunteers into two occasions, one where time >22 hours post dose and time ≤22 hours post dose. Hence the 3 visit numbers 2, 3 and 4 in healthy volunteers where treated as occasions 1, 2, 3, 4, 5, 6. These allowed the model to predict all data without affecting the estimates of cortisol PK. Whether circadian rhythm in Al patients exists, is not known. Regardless, the putative contribution from a circadian from Al patients and healthy volunteers is likely to be minor to the overall cortisol profile.

The final PK model described both healthy volunteers and patient data well. All dose levels were equally well predicted as well as different body weight ranges. No difference was found between the two assays used in determining hydrocortisone (cortisol) concentrations in Phase I and Phase II/III for unfed data. The cortisol versus time profile in a typical patient with a body weight of 80 kg after a total daily dose of 20, 30 and 40 mg Hydrocortisone (composition according to reference example 1) and conventional hydrocortisone tablets (Hydrocortone, MSD) t.i.d. is shown in Fig. 1.

### Results

### Population PK modelling of conventional hydrocortisone tablet

A two-compartmental model with first-order elimination and absorption with an absorption lag-time described the data adequately. The endogenous baseline levels were modelled as described for Hydrocortisone (composition according to reference example 1) above. The bioavailability was found to decrease nonlinearly with increasing oral doses. Oral clearance (CL/F) was found to increase linearly with body weight. The relationship between weight and CL/F has been reported earlier and a weight nomogram for hydrocortisone tablets has been described (Mah et al. 2004). No significant relation between Hydrocortisone (composition according to reference example 1) PK and age, renal function (MDRD) or gender was supported in the POPPK analysis. The hydrocortisone versus time profile in a typical patient with 80 kg after a total daily dose of 20, 30 and 40 mg is shown in Fig. 1. The maximal concentration during the day after the same total daily dose is predicted to be approximately similar for Hydrocortisone (composition according to reference example 1) and hydrocortisone tablets. However, the total AUC is lower after Hydrocortisone (composition according to reference example 1) compared to the same dose of the hydrocortisone tablets, which is most likely due to the lower bioavailability after Hydrocortisone (composition according to reference example 1) compared to hydrocortisone tablets. However, a lower total AUC after Hydrocortisone (composition according to reference example 1) is not regarded as a clinical disadvantage since the higher AUC after tablets is mainly reflected in the two additional peaks during the days. Exactly these high peaks during the day are thought to be related to glucocorticoid associated side effects since these peaks are not seen in the healthy circadian cortisol profile (Fig. 2). The lack of fluctuation during the day after Hydrocortisone (composition according to reference example 1) in contrast to after hydrocortisone tablets is therefore of clinical benefits.

### Weight Based Nomogram

The derived AUC ratios (exposure at each weight level/target exposure) based on typical parameters and different doses are shown in Fig. 10. A ratio of 1 indicates that the dose will result in the target exposure. A ratio <1 indicates lower exposure compared to target. A ratio >1 indicates a higher exposure than target. Yellow cells indicate the selected dose level for each weight span. For the lowest dose of 15 mg and a weight range of 55-59 kg, the lowest weight, 55 kg, will result in a 7% higher (ratio=1.07) exposure than the target. The highest weight within the same weight band, 59 kg, will result in a 2% lower (ratio=0.98) exposure than the target. The corresponding exposure ranges for the 20, 25, 30, 35 and 40 mg dose levels were 1.16-0.99, 1.09-0.95, 1.00-0.95, 0.97-0.93 and 0.94-0.83. The lowest weight in the nomogram, 50 kg, was set by the weight distribution of data included in the model (minimum weight= 53 kg). The highest weight in the nomogram, 100 kg, was set where the deviation from the target was at its maximum (ratio=0.83). The exposure was simulated with only typical parameters. Therefore, the deviations from the target are what are predicted on average based on a weight. The final weight nomogram is shown in Table 1. Due to the nonlinearity in bioavailability, the increase in dose from 30 to 35 mg is compensated to a large degree by the nonlinear decrease in bioavailability. Due to the linear relationship between oral clearance and weight, the 35 mg dose does not provide sufficient exposure in the weight span 85-89 kg (ratio below 1). A 40 mg dose for this weight span would be indicated given the simulations and would result in the weight nomogram not including the 35 mg dose. However, due to safety reasons and in order to include a 35 mg dose, the 35 and 40 mg doses were selected for the 85-89 kg and ≥90 kg weight span, respectively.

There was a good agreement between the dose derived from the weight nomogram and the individualised dose used in the patient study which was based on clinical best practice. The average percent difference [(AUC ₚₕₐₛₑ II/III -AUC _{nomogram})/ AUC phase _{II/III}] on an individual level between the two regimens was only 1.7%. Fewer patients were exposed to very high cortisol levels with a dose based on the weight nomogram compared to clinical best practise. The 10-90% quantiles of AUC after the clinical doses and weight nomogram were 1735-4795 h*nM and 1670-3795 h*nM, respectively. The between patient variability in total AUC was slightly lower (CV=35%) after weight nomogram doses as compared to the total AUC after doses used in the Phase II/III study (CV=37%). The distribution of individual AUC using the weight nomogram compared to clinical best practise is shown in Fig. 3. The median cortisol exposure (AUC) due to drug administration was predicted to be 2840 h*nM and 2540 h*nM after doses based on clinical best practice and weight nomogram, respectively. If all patients had been given 30 mg, the median exposure was predicted to be 2685 h*nM. Hence, the on an average slightly lower exposure after the weight nomogram compared to the clinical doses is not judged to be of a disadvantage since simulations showed that the exposure after the weight nomogram was in agreement with exposure in healthy volunteers and that the average exposure after the weight nomogram was similar as to the average exposure of an non-individualised 30 mg standard dose. Exposure after 30 mg (composition according to reference example 1) in a typical patient was defined as the clinical target. The 10-90% percentile in exposure after a standard dose was 1810-4555 h*nM with a CV of 37%. The improvements in benefit of a weight nomogram compared to a standard dose of 30 mg without any dose individualisation is somewhat difficult to evaluate when just looking at the patients in clinical study since according to the weight distribution and the nomogram, 27% of patients should have a dose of 30 mg.

The results show that the weight nomogram can improve individualised dosing in Al patients without residual endogenous cortisol concentrations or who have endogenous cortisol concentrations as low as ≤ 150 nM. However, a PK nomogram will further improve the individualisation of dosing due to between patient variability in oral clearance and remaining endogenous secretion that may occur in some patients.

### Development of PK nomogram

### Evaluation of optimal sampling times for PK nomogram

The simulation of optimal sampling times for a PK nomogram revealed that the highest precision in CL/F was using a pre-dose sample together with a 6-hour post-dose sample. The CV was 11% using a 6-hour sample in comparison to 25, 17 and 15% in CV for a 2-, 4- and 8-hour post-dose sample (Fig. 9).

### Estimation of cortisol concentration cut-off values for the PK nomogram

Two different sets of CL cut-off values for the different doses in the PK nomogram were evaluated. One set included CL cut-off values where the ratio for the highest dose 40 mg was 0.85, i.e., a 15% lower exposure target (CL=24.21146). The CL cut-off value was then set to a value between the cut-off for the unadjusted 40 and 30 mg dose (CL=21.3750).

In order to evaluate which of the sets of CL cut-off values to be used in the PK nomogram, a Ccortisol value from each patient in the Phase II/III study after a 20 mg DuoCort dose was predicted and a dose based on the PK nomogram derived using adjusted and non-adjusted cut-offs for the different doses.

The final PK nomogram was based on adjusted CL-cut-off values (Fig. 11) where higher doses were given lower weight in the PK nomogram due to safety reasons as well as based on diagnostics. The individual patient's CL at first occasion based on the final DuoCort PK model and the corresponding C_{cortisol} after a 20 mg test dose is shown in Figure 13a. These Ccortisol values are predicted as if the PK nomogram was to be used for these patients. There was a nice predicted relationship between CL and Ccortisol. Based on these Ccortisol values and the final PK nomogram, individual doses were derived. The corresponding AUCs based on the doses from the PK nomogram were thereafter derived (Figure 13b). As can be seen in Figure 13c, no trends were seen in AUC versus CL indicating that regardless of CL, the PK nomogram resulted in a relevant exposure.

There was a very good agreement between the dose derived from the PK nomogram and the individualised dose used in the patient study which was based on clinical practice (Fig. 4). The average percent difference [(AUC phase II/III -AUC nomogram)/ AUC phase II/III] on an individual level between the two regimens was only 0.34%. The median in AUC when administration was predicted by the PK nomogram was 2620 h*nM which was close to the medians after weight nomogram (2540 h*nM) and standard dose of 30 mg (2685 h*nM). Fewer patients were exposed to very high cortisol levels with a dose based on the weight nomogram compared to clinical practise. The 10th-90th interquantile range in AUC was 1766-3388 h*nM using the PK nomogram. The CV in AUC was 25.7% after the PK nomogram in comparison to 37% and 35% when doses based on clinical practice or weight nomogram were used. Hence, the PK nomogram decreased the between patient variability in exposure compared to both doses based on clinical practice and the weight nomogram. The simulated 90% prediction interval in AUC after doses based on the PK nomogram is shown in Fig. 14 showing the good agreement between the exposure after the dose derived from the PK nomogram and the individualised dose used in the patient study which was based on clinical practice.

When CL cut-off values were used, where the ratio to target was as close to 1 as possible for all doses (see Appendix N), trends were seen in AUC versus CL as well as AUC versus dose (Fig. 15). Higher AUC was seen for the highest 40 mg doses and lower CL in the interval (close to 20 L/h) indicating a too low CL cut-off value for the highest dose (40 mg).

IOV reduces the power of a single plasma concentration to predict CUF and thereby AUC. However, IOV was estimated to be less than IIV for CL/F wherefore a PK nomogram still is powerful. IOV was estimated to be larger than IIV for central volume of distribution but this will have minor impact at a 6-hour sample where IOV in CL/F will dominate. In order to evaluate the impact of IOV on the PK nomogram, the CV in AUC when only one test dose was given was compared to perfect information which would be if one could give a test dose before each dosing occasion (which is impossible in practice). When the dose was individualised based on a test dose of 20 mg at each occasion, the CV in AUC was 19.4 % as compared to 25.7% for one occasion. The decrease in CV in AUC using a PK nomogram and one dose individualisation occasion was an improvement compared to the doses given by clinical practice and the weight nomogram wherefore the additional gain in using more than one dose individualisation occasion was judged to be minor compared to the unpractical issues. Only one sample occasion is suggested.

### Dosage recommendation based upon PK measurements

The highest precision in CUF was found using a 6 hours sample as compared to 2, 4 or 8 hours. Pre-dose cortisol levels were estimated in 15% of the patients in study DC 06/02 Part A indicating some remaining endogenous cortisol production. Two blood samples need to be drawn to derive the dose from the PK nomogram in order to account for putative pre-dose cortisol levels. Therefore, the procedure for deriving an individual dose based on the PK nomogram is as follows:
a A blood sample is taken pre-dose from the subject in fasted state.
b An oral test dose of 20 mg hydrocortisone (composition according to reference example 1) is given. This should be the first dose of hydrocortisone administered during that particular day
c Exactly 6 hours post-dose, a second blood sample is withdrawn
d The serum cortisol concentration is determined in the two samples by an immunoassay method.
e The difference in serum cortisol concentration (C_{cortisol}= C₆ₕ -C₀ₕ) between 6 hours post-dose (C₆ₕ) and pre-dose (C₀ₕ) is derived
f The individual once daily hydrocortisone (composition according to reference example 1) dose is derived based on the derived C_{cortisol} and C₀ₕ values and the PK nomogram according to the invention.

The C_{cortisol} value will be an indirect measure of the individual oral clearance. Depending on the level of endogenous cortisol level, the dose might be adjusted compared to patients without endogenous cortisol levels. The PK nomogram gives doses for patients with residual endogenous cortisol levels in the range 150-250 nM and 250-350 nM. These doses are lower than doses suggested for patients with endogenous levels below 150 nM given the same oral clearance (C_{cortisol}). The endogenous serum cortisol concentration intervals suggested in the PK nomogram are based on that all but one Addison patients in clinical trials had baseline cortisol levels below 150 nM and that the value of cortisol replacement in individuals with levels above 350 nM is most likely small. The range in baseline endogen cortisol levels in patients with partial insufficiency is most likely to be below 350 nM in most individuals (Agha et al. 2004). The serum cortisol concentrations for endogenous interval suggested in the PK nomogram are also of clinical significance. Patents with early morning serum cortisol concentration (7-9 AM) below 100 nmol/l have most likely adrenal insufficiency and any further testing or evaluation is often not necessary if the clinical picture is clear. On the other hand, patients who have morning serum levels between approximately 100 and 350 nmol/l will need further testing and clinical evaluation before it can be determined whether they need daily life-long glucocorticoid replacement therapy.

The median in AUC predicted by the PK nomogram due to drug administration was 2620 h*nM. The 10-90% interquantile range in AUC was 1766-3388 h*nM using the PK nomogram. The CV in AUC was 26% after the PK nomogram in comparison to 37% and 35% when doses based on clinical best practice or weight nomogram was used. Hence, the PK nomogram decreased the between patient variability in exposure compared to both doses based on clinical best practice and the weight nomogram (Fig. 4).

IOV reduces the power for a single plasma concentration to predict CUF and thereby AUC. However, IOV was estimated to be less than IIV for CL/F wherefore a PK nomogram still is powerful. IOV was estimated to be larger than IIV for central volume of distribution but this will have minor impact at a 6 hr sample where IOV in CL/F will dominate. In order to evaluate the impact of IOV on the PK nomogram, the CV in AUC when only one test dose was given was compared to perfect information which would be if one could give a test dose at each occasion. When the dose was individualized based on a test dose of 20 mg at each occasion, the CV in AUC was 19.4 % as compared to 25.7% for one occasion. The decrease in CV in AUC using a PK nomogram and one dose individualization occasion was an improvement compared to the doses given by clinical best practice and the weight nomogram wherefore a the additional gain in using more than one dose individualization occasion was judged to be minor compared to the unpractical issues. Only one sample occasion is suggested.

Only one patient in the clinical study had endogenous cortisol levels above 150 nM. Hence, the accuracy of the PK nomogram for patient with residual endogenous cortisol levels above 150 nM could not be validated using observed data. Instead a typical patient for each endogenous cortisol interval and C_{cortisol} lower limit was simulated and the cortisol profile was compared visually to a typical patient profile with endogenous cortisol levels (40 nM) (Fig. 5). For each of the Cₒᵣₜᵢₛₒₗ lower limits in each of two intervals 150-250 nM (Fig. 6) and 250-350 nM (Fig. 7), there was a good agreement between cortisol profiles after adjusted doses for patients with endogenous cortisol levels and profiles in non-adjusted doses in patients with endogenous cortisol levels ≤150 nM (Fig. 5).

### Evaluation of optimal sampling times for PK nomograms

This was done by using observations from one rich sample occasion in the 62 patients in a Phase II/III study. The final PK model was used to re-estimate the individual PK parameters based on the final PK model for these different situations:
- All observed PK sampling points
- Pre-dose plus at 2 hours post-dose
- Pre-dose plus at 4 hours post-dose
- Pre-dose plus at 6 hours post-dose
- Pre-dose plus at 8 hours post-dose

The individual Bayes estimates for CL/F of patients using all observed PK sampling points was used as reference value (CLₜᵣᵤₑ). For each individual and situation above, CLₜᵣᵤₑ/CLₓₕᵣ was derived, wherein the x stands for the hours passed post-dose as seen above (2 hours, 4 hours, 6 hours and 8 hours). The precision in CL/F for each sampling time point (2, 4, 6 or 8 hours) was calculated as the coefficient of variation (CV) of all CL_{frue}/CLₓₕᵣ values. A ratio of CLₜᵣᵤₑ/CLₓₕᵣ = 1 would indicate that a sample at x hours would be as good as a full rich PK profile. The optimal sampling time was found to be 6 hours post-dose as the coefficient of variation (CV) was found to be the lowest at this interval and amounted to 11% (cf. Fig. 9)

### Dosage recommendation during intercurrent illness

The phase II/III study and the phase IIIB study provided safety information about intercurrent illness regimens of doubling the maintenance dose from once daily to twice daily. Pharmacometric simulations can therefore provide additional information needed to suggest intercurrent illness regimens.

Simulations of intercurrent illness regimens showed that giving an additional dose at 8±2 hours after the first morning dose resulted in slightly higher peak concentrations for the second dose but still fairly low concentrations during the night (Fig. 8a-c). This regimen is therefore recommended for milder clinical conditions, e.g., minor surgery or low-grade infection. Giving three daily doses of Hydrocortisone (composition according to reference example 1) with 8 hours intervals resulted in no accumulation after the third daily dose compared to the second and with cortisol levels during the night as well (Fig. 8d). This regimen is therefore recommended in more serious conditions. Increasing the oral dose but keeping the o.d. regimen does not provide sufficient cortisol coverage in intercurrent illness conditions (Fig. 8e). The simulations of intercurrent illness was made with 30 mg but the conclusions are valid for the other dose levels as well since the accumulation and time to steady state is only dependent on the terminal half-life which is dose-independent.

### References

Vgontzas AN, Bixler EO, Lin HM, Prolo P, Mastorakos G, Vela-Bueno A, Kales A, Chrousos GP. Chronic insomnia is associated with nyctohemeral activation of the hypothalamic-pituitary-adrenal axis: clinical implications. J Clin Endocrinol Metab. 2001;86(8):3787-3794
Mah PM, Jenkins RC, Rostami-Hodjegan A, Newell-Price J, Doane A, Ibbotson V, et al. Weight-related dosing, timing and monitoring hydrocortisone replacement therapy in patients with adrenal insufficiency. Clin Endocrinol (Oxf). 2004;61(3):367-375
Agha A, Liew A, Finucane F, Baker L, O'Kelly P, Tormey W, Thompson CJ. Conventional glucocorticoid replacement overtreats adult hypopituitary patients with partial ACTH deficiency. Clin Endocrinol (Oxf). 2004;60(6):688-93
Beal SL, Sheiner LB, Boeckmann AJ. NONMEM users guides. ICON Development Solutions, Ellicott City, MD, 1989-2006, http://www.globomaxnm.com/nonmem.htm
Lennernäs H, Skrtic S, Johannsson G. Replacement therapy of oral hydrocortisone in adrenal insufficiency: the influence of gastrointestinal factors. Expert Opin Drug Metab Toxicol. 2008;4(6):749-758
Axelrod L. Glucocorticoid therapy. Endocrinology. L. J. DeGrooth and J. C. Jameson. Philadelphia, Elsevier Saunders. 2006;5: 2329-42.
Amidon GL, Lennernäs H, Shah VP, Crison JR. A theoretical basis for a biopharmaceutic drug classification: the correlation of in vitro drug product dissolution and in vivo bioavailability. Pharm Res. 1995;12:413-20.
Hong Y, Mager DE, Blum RA, Jusko WJ. Population pharmacokinetic/pharmacodynamic modeling of systemic corticosteroid inhibition of whole blood lymphocytes: modeling interoccasion pharmacodynamic variability. Pharm Res. 2007;24(6):1088-97.
Liapi and Chrousos, 1992. Glucocorticoids. In: Jaffe SJ, Aranda JV (eds) Pediatric Pharmacology, 2nd Edition, WB Saunders Co, Philadelphia, pp. 466-75.
White, K et al., Adrenal crisis in treated Addison's disease: a predictable but under-managed event. Eur, J. Endocrinology, 2010, 162, pp. 115-120

## Claims

1. A composition for use in glucocorticoid replacement therapy by oral administration of the composition once daily in the morning according to a pharmacokinetic (PK) nomogram, as follows;
| | C₀ₕ ≤150 nM | 150<C₀ₕ≤250 nM | 250< C₀ₕ ≤350 nM |
|---|---|---|---|
| Cₒᵣₜᵢₛₒₗ (nM) | Dose (mg) | Dose (mg) | Dose (mg) |
| ≥198 | 15 | 10 | 5 |
| 197-161 | 20 | 10 | 5 |
| 160-141 | 25 | 15 | 10 |
| 140-115 | 30 | 15 | 10 |
| 114-97 | 35 | 15 | 10 |
| <97 | 40 | 15 | 10 |
wherein the dose is the total daily dose of hydrocortisone to be administered and wherein the C_{cortisol}(nM) is the difference in serum cortisol concentration between 6 hours post-dose and pre-dose as defined herein;
wherein the composition is a dual composition which comprises an immediate release part and an extended release part, and wherein the composition is prepared using a composition according to the table below
| | |
|---|---|
| Glucocorticoid | 1.5 wt% to 6.5 wt% |
| Polymer, Binder | 15 wt% to 25 wt% |
| Filler | 30 wt% to 40 wt% |
| Glidant | 0.1 wt% to 0.5 wt% |
| Lubricant | 0.1 wt% to 0.5 wt% |
| Film Coating System | 1 wt% to 5 wt% |
| Solvent | 25 wt% to 40 wt% |

2. A composition for use according to any of the preceding claims, wherein the ratio of the glucocorticoids in the composition between the immediate release part and the extended release part is in range from about 1:1 to about 0.01:1.

3. A composition for use according to any of the preceding claims, wherein the composition is administered in form of an oral dosage, wherein from about 15 to about 35% w/w of the total amount of the glucocorticoids is immediate released upon administration and the remaining part of the glucocorticoids is modified released during a time period of at least about 8 hours such as at least about 12 hours.

4. A composition for use according to any of the preceding claims, wherein the one or more glucocorticoids is/are administered in form of a single-unit dosage form comprising a core containing the extended release part of the glucocorticoids and the core being coated with the remaining part of the glucocorticoid and wherein the coating is the immediate release part.

5. A composition for use according to any of the preceding claims, wherein the administration of the once daily dose is supplemented with one or more further doses of glucocorticoids and may further be twice daily or 3 times daily or 4 times daily or 5 times daily or 6 times daily.

6. A composition for use according to claim 5, wherein the administration is oral or parenteral.

7. A composition for use according to any of the preceding claims, wherein the time interval between any consecutive administrations is e.g. about 2 hours or more, such as e.g. about 3 hours or more, such as e.g. about 4 hours or more, such as e.g. about 5 hours or more, such as e.g. about 6 hours or more, such as e.g. about 7 hours or more, such as e.g. about 8 hours or more, such as e.g. about 9 hours or more, such as e.g. about 10 hours or more, such as e.g. about 11 hours or more, such as e.g. about 12 hours or more independently of each other.

8. A composition for use according to any of the preceding claims, wherein the total daily dose of glucocorticoids is e.g. about 15 mg or about 20 mg or about 25 mg or about 30 mg or about 40 mg or about 50 mg or about 60 mg or about 70 mg or about 80 mg or about 90 mg or about 100 mg or about 150 mg or about 200 mg.

9. A composition for use according to any of the preceding claims, wherein the administration can be any combination of different unit dosages such as e.g. a combination of any number of dosage forms containing a 5 mg dose and/or any number of dosage forms containing a 20 mg dose.

10. A composition for use according to any of the preceding claims, wherein the composition is in form of a solid dosage form including a tablet or a capsule.

11. A composition for use according to any of the preceding claims wherein the composition is according to the table below
| **Ingredient** | **Quantity (5 mg tablet), mg/unit** | **Quantity (20 mg tablet), mg/unit** | **Standard** |
|---|---|---|---|
| Hydrocortisone | 5.0 | 20.0 | Ph.Eur. |
| Hypromellose K 100 cP (Methocel K 100) | 47.05 | 41.2 | Ph.Eur. |
| Hypromellose K 4000 cP (Methocel K4M) | 20.0 | 24.6 | Ph.Eur. |
| Cellulose, microcrystalline (Avicel PH-102) | 100.8 | 100.8 | Ph.Eur. |
| Starch, pregelatinized (Starch 1500) | 16.4 | 16.4 | Ph.Eur. |
| Silica colloidal anhydrous (Aerosil 200) | 1.0 | 1.0 | Ph.Eur. |
| Magnesium stearate | 1.0 | 1.0 | Ph.Eur. |
| Opadry II | about 13.75 | about 11.0 | Colorcon |
| Water, purified* | about 102 | about 107 | Ph.Eur. |
| | | | |
|---|---|---|---|
| *Evaporates during the manufacturing process | | | |
wherein the composition is in the form of a single unit dosage form comprising a core containing the extended release part, the core being coated wherein the coating is the immediate release part;
and in the case of a 5 mg tablet an amount of 1.25 mg of hydrocortisone is in the coating and 3.75 mg of hydrocortisone is in the core, and in the case of a 20 mg tablet the coating has an amount of 5 mg of hydrocortisone and an amount of 15 mg of hydrocortisone in the core.

12. A method for deriving an individualized dosing of glucocorticoids, the method comprising;
(i) determining, by an immunoassay method, the serum cortisol concentration C₀ₕ of a pre-dose blood sample, wherein the pre-dose blood sample is a sample prior to administering a dose of a glucocorticoid to a subject in a fasted state,
(ii) determining, by an immunoassay method, the serum cortisol concentration C₆ₕ of a post-dose blood sample, wherein the post-dose blood sample is a sample at exactly 6 hours post-dose of a 20 mg hydrocortisone or hydrocortisone equivalent oral test dose, and wherein the oral test dose was the first dose of hydrocortisone or hydrocortisone equivalent during that particular day,
(iii) deriving the difference in serum cortisol concentration (C_{cordsol} = C₆ₕ-C₀ₕ) between 6 hours post-dose (C₆ₕ) and pre-dose (C₀ₕ),
(iv) deriving the individual once daily hydrocortisone or hydrocortisone equivalent dose based on the derived C_{cortisol} and C₀ₕ values and the PK nomogram according to the table below
| | C₀ₕ ≤150 nM | 150< C₀ₕ ≤250 nM | 250< C₀ₕ ≤350 nM |
|---|---|---|---|
| C_{cortisol} (nM) | Dose (mg) | Dose (mg) | Dose (mg) |
| ≥198 | 15 | 10 | 5 |
| 197-161 | 20 | 10 | 5 |
| 160-141 | 25 | 15 | 10 |
| 140-115 | 30 | 15 | 10 |
| 114-97 | 35 | 15 | 10 |
| <97 | 40 | 15 | 10 |
wherein the oral test dose is a dual composition which comprises an immediate release part and an extended release part, and wherein the oral test dose is prepared using a composition according to the table below
| | |
|---|---|
| Glucocorticoid | 1.5 wt% to 6.5 wt% |
| Polymer, Binder | 15 wt% to 25 wt% |
| Filler | 30 wt% to 40 wt% |
| Glidant | 0.1 wt% to 0.5 wt% |
| Lubricant | 0.1 wt% to 0.5 wt% |
| Film Coating System | 1 wt% to 5 wt% |
| Solvent | 25 wt% to 40 wt% |

13. A method according to claim 12, wherein the oral test dose is according to the table below
| **Ingredient** | **Quantity (5 mg tablet), mg/unit** | **Quantity (20 mg tablet), mg/unit** | **Standard** |
|---|---|---|---|
| Hydrocortisone | 5.0 | 20.0 | Ph.Eur. |
| Hypromellose K 100 cP (Methocel K 100) | 47.05 | 41.2 | Ph.Eur. |
| Hypromellose K 4000 cP (Methocel K4M) | 20.0 | 24.6 | Ph.Eur. |
| Cellulose, microcrystalline (Avicel PH-102) | 100.8 | 100.8 | Ph.Eur. |
| Starch, pregelatinized (Starch 1500) | 16.4 | 16.4 | Ph.Eur. |
| Silica colloidal anhydrous (Aerosil 200) | 1.0 | 1.0 | Ph.Eur. |
| Magnesium stearate | 1.0 | 1.0 | Ph.Eur. |
| Opadry II | about 13.75 | about 11.0 | Colorcon |
| Water, purified* | about 102 | about 107 | Ph.Eur. |
and the oral test dose is in the form of a single unit dosage form comprising a core containing the extended release part, the core being coated wherein the coating is the immediate release part; and in the case of a 5 mg tablet an amount of 1.25 mg of hydrocortisone is in the coating and 3.75 mg of hydrocortisone is in the core, and in the case of a 20 mg tablet the coating has an amount of 5 mg of hydrocortisone and an amount of 15 mg of hydrocortisone in the core.

14. A composition for use according to any of claims 1-9, wherein the composition is administered according to a weight nomogram, as follows:
| Dose (mg) | Body weight (kg) |
|---|---|
| 15 | 55-59 |
| 20 | 60-69 |
| 25 | 70-79 |
| 30 | 80-84 |
| 35 | 85-89 |
| 40 | 90-100 |
and wherein the dose is the total daily dose of hydrocortisone to be administered and the body weight is the bodyweight of the subject.

## Patentansprüche

1. Zusammensetzung zur Verwendung in der Glukokortikoid-Ersatztherapie durch einmal tägliche morgendliche orale Verabreichung der Zusammensetzung nach einem pharmakokinetischen (PK) Nomogramm, wie folgt:
| | C₀ₕ≤150 nM | 150<C₀ₕ ≤250 nM | 250< C₀ₕ ≤350 nM |
|---|---|---|---|
| C_{cortisol} (nm) | Dosis (mg) | Dosis (mg) | Dosis (mg) |
| >198 | 15 | 10 | 5 |
| 197-161 | 20 | 10 | 5 |
| 160-141 | 25 | 15 | 10 |
| 140-115 | 30 | 15 | 10 |
| 114-97 | 35 | 15 | 10 |
| <97 | 40 | 15 | 10 |
wobei die Dosis die zu verabreichende tägliche Gesamtdosis von Hydrokortison ist und wobei (C_{cortisol} (nM) die Differenz in der Serumcortisolkonzentration zwischen 6 Stunden nach der Verabreichung und vor der Verabreichung wie hierin definiert ist;
wobei die Zusammensetzung eine duale Zusammensetzung ist, die einen Teil mit sofortiger Freisetzung und einen Teil mit verlängerter Freisetzung umfasst, und wobei die Zusammensetzung unter Verwendung einer Zusammensetzung gemäß der nachfolgenden Tabelle hergestellt wird
| | |
|---|---|
| Glukokortikoid | 1,5 Gew.-% bis 6,5 Gew.-% |
| Polymer, Bindemittel | 15 Gew.-% bis 25 Gew.-% |
| Füllstoff | 30 Gew.-% bis 40 Gew.-% |
| Gleitmittel | 0,1 Gew.-% bis 0,5 Gew.-% |
| Schmiermittel | 0,1 Gew.-% bis 0,5 Gew.-% |
| Filmbeschichtungssystem | 1 Gew.-% bis 5 Gew.-% |
| Lösungsmittel | 25 Gew.-% bis 40 Gew.-% |

2. Zusammensetzung zur Verwendung nach einem der vorhergehenden Ansprüche, wobei das Verhältnis der Glukokortikoide in der Zusammensetzung zwischen dem Teil mit sofortiger Freisetzung und dem Teil mit verlängerter Freisetzung im Bereich von ungefähr 1:1 und ungefähr 0,01:1 liegt.

3. Zusammensetzung zur Verwendung nach einem der vorhergehenden Ansprüche, wobei die Zusammensetzung in Form einer oralen Dosierung verabreicht wird, wobei ungefähr 15 bis ungefähr 35% g/g der Gesamtmenge der Glukokortikoide bei Verabreichung sofort freigesetzt werden und der restliche Teil der Glukokortikoide während einer Zeitspanne von wenigstens ungefähr 8 Stunden, wie etwa wenigstens ungefähr 12 Stunden, modifiziert freigesetzt wird.

4. Zusammensetzung zur Verwendung nach einem der vorhergehenden Ansprüche, wobei das eine oder die mehreren Glukortikoid/e in Form einer Einzeleinheitsdosisform, die einen Kern umfasst, der den Teil der Glukokortikoide mit verlängerter Freisetzung enthält, verabreicht wird/werden, wobei der Kern mit dem restlichen Glukortikoidteil beschichtet ist, und wobei die Beschichtung der Teil mit der sofortigen Freisetzung ist.

5. Zusammensetzung zur Verwendung nach einem der vorhergehenden Ansprüche, wobei die Verabreichung der einmal täglichen Dosis durch eine oder mehrere weitere Glukokortikoiddosen ergänzt wird und ferner zweimal täglich oder 3 Mal täglich oder 4 Mal täglich oder 5 Mal täglich oder 6 Mal täglich erfolgen kann.

6. Zusammensetzung zur Verwendung nach Anspruch 5, wobei die Verabreichung oral oder parenteral erfolgt.

7. Zusammensetzung zur Verwendung nach einem der vorhergehenden Ansprüche, wobei die Zeitspanne zwischen beliebigen aufeinanderfolgenden Verabreichungen z.B. ungefähr 2 Stunden oder mehr, wie etwa z.B. ungefähr 3 Stunden oder mehr, wie etwa z.B. ungefähr 4 Stunden oder mehr, wie etwa z.B. ungefähr 5 Stunden oder mehr, wie etwa z.B. ungefähr 6 Stunden oder mehr, wie etwa z.B. ungefähr 7 Stunden oder mehr, wie etwa z.B. ungefähr 8 Stunden oder mehr, wie etwa z.B. ungefähr 9 Stunden oder mehr, wie etwa z.B. ungefähr 10 Stunden oder mehr, wie etwa z.B. ungefähr 11 Stunden oder mehr, wie etwa z.B. ungefähr 12 Stunden oder mehr, unabhängig voneinander beträgt.

8. Zusammensetzung zur Verwendung nach einem der vorhergehenden Ansprüche, wobei die tägliche Gesamtdosis der Glukokortikoide z.B. ungefähr 15 mg oder ungefähr 20 mg oder ungefähr 25 mg oder ungefähr 30 mg oder ungefähr 40 mg oder ungefähr 50 mg oder ungefähr 60 mg oder ungefähr 70 mg oder ungefähr 80 mg oder ungefähr 90 mg oder ungefähr 100 mg oder ungefähr 150 mg oder ungefähr 200 mg beträgt.

9. Zusammensetzung zur Verwendung nach einem der vorhergehenden Ansprüche, wobei die Verabreichung eine beliebige Kombination von verschiedenen Einheitsdosen sein kann, wie etwa z.B. eine Kombination einer beliebigen Anzahl von Darreichungsformen, die eine Dosis von 5 mg enthalten, und/oder eine beliebige Anzahl von Darreichungsformen, die eine Dosis von 20 mg enthalten.

10. Zusammensetzung zur Verwendung nach einem der vorhergehenden Ansprüche, wobei die Zusammensetzung in Form einer festen Darreichungsform vorliegt, die eine Tablette oder Kapsel aufweist.

11. Zusammensetzung zur Verwendung nach einem der vorhergehenden Ansprüche, wobei die Zusammensetzung der nachfolgenden Tabelle entspricht
| Inhaltsstoff | Menge (5 mg Tablette), mg/Einheit | Menge (20 mg Tablette), mg/Einheit | Standard |
|---|---|---|---|
| Hydrokortison | 5, 0 | 20,0 | Ph.Eur. |
| Hypromellose K 100 cP (Methocel K 100) | 47,05 | 41,2 | Ph.Eur. |
| Hypromellose K 4000 cP (Methocel K4M) | 20,0 | 24,6 | Ph.Eur. |
| Mikrokristalline Cellulose (Avicel PH-102) | 100,8 | 100,8 | Ph.Eur. |
| Vorgelatinisierte Stärke (Stärke 1500) | 16,4 | 16,4 | Ph.Eur. |
| Hochdisperses Siliciumdioxid (Aerosil 200) | 1,0 | 1,0 | Ph.Eur. |
| Magnesiumstearat | 1,0 | 1,0 | Ph.Eur. |
| Opadry II | Ca. 13,75 | Ca. 11,0 | Colorcon |
| Gereinigtes Wasser* | Ca. 102 | Ca. 107 | Ph.Eur. |
| | | | |
|---|---|---|---|
| * Verdunstet während des Herstellungsverfahrens | | | |
wobei die Zusammensetzung in Form einer Einzeleinheitsdosisform vorliegt, die einen Kern umfasst, der den Teil mit verlängerter Freisetzung enthält, wobei der Kern beschichtet ist, wobei die Beschichtung der Teil mit sofortiger Freisetzung ist; und im Fall einer 5-mg-Tablette eine Menge von 1,25 mg Hydrokortison in der Beschichtung vorliegt und 3,75 mg Hydrokortison im Kern vorliegt, und im Fall einer 20-mg-Tablette die Beschichtung eine Menge von 5 mg Hydrokortison aufweist und eine Menge von 15 mg Hydrokortison im Kern vorliegt.

12. Verfahren zur Ableitung einer individualisierten Dosierung von Glukokortikoiden, wobei das Verfahren Folgendes umfasst:
(i) Bestimmen, mittels eines Immunassay-Verfahrens, der Serumcortisolkonzentration C₀ₕ einer vor Verabreichung entnommenen Blutprobe, wobei die vor Verabreichung entnommene Blutprobe eine Probe vor Verabreichung einer Dosis eines Glukokortikoids an einen Patienten in einem Nüchternzustand ist,
(ii) Bestimmen, mittels eines Immunassay-Verfahrens, der Serumcortisolkonzentration C₆ₕ einer nach Verabreichung entnommenen Blutprobe, wobei die nach Verabreichung entnommene Blutprobe eine Probe ist, die exakt 6 Stunden nach Verabreichung einer oralen Testdosis von 20 mg Hydrokortison oder eines Hydrokortison-Äquivalents entnommen wurde, und wobei die orale Testdosis die erste Dosis von Hydrokortison oder des Hydrokortison-Äquivalents während dieses jeweiligen Tages war,
(iii) Ableiten der Differenz in der Serumcortisolkonzentration (C_{cortisol} = C₆ₕ-C₀ₕ) zwischen 6 Stunden nach Verabreichung (C₆ₕ) und vor Verabreichung (C₀ₕ),
(iv) Ableiten der individuellen einmal täglichen Dosis von Hydrokortison oder eines Hydrokortison-Äquivalents auf Basis der abgeleiteten C_{cortisol}- und C₀ₕ-Werte und des PK-Nomogramms gemäß der nachfolgenden Tabelle:
| | C₀ₕ≤150 nM | 150<C₀ₕ ≤250 nM | 250< C₀ₕ ≤350 nM |
|---|---|---|---|
| C_{cortisol} (nm) | Dosis (mg) | Dosis (mg) | Dosis (mg) |
| >198 | 15 | 10 | 5 |
| 197-161 | 20 | 10 | 5 |
| 160-141 | 25 | 15 | 10 |
| 140-115 | 30 | 15 | 10 |
| 114-97 | 35 | 15 | 10 |
| <97 | 40 | 15 | 10 |
wobei die orale Testdosis eine duale Zusammensetzung ist, die einen Teil mit sofortiger Freisetzung und einen Teil mit verlängerter Freisetzung umfasst, und wobei die orale Testdosis unter Verwendung einer Zusammensetzung gemäß der nachfolgenden Tabelle hergestellt wird
| | |
|---|---|
| Glukokortikoid | 1,5 Gew.-% bis 6,5 Gew.-% |
| Polymer, Bindemittel | 15 Gew.-% bis 25 Gew.-% |
| Füllstoff | 30 Gew.-% bis 40 Gew.-% |
| Gleitmittel | 0,1 Gew.-% bis 0,5 Gew.-% |
| Schmiermittel | 0,1 Gew.-% bis 0,5 Gew.-% |
| Filmbeschichtungssystem | 1 Gew.-% bis 5 Gew.-% |
| Lösungsmittel | 25 Gew.-% bis 40 Gew.-% |

13. Verfahren nach Anspruch 12, wobei die orale Testdosis der nachfolgenden Tabelle entspricht
| Inhaltsstoff | Menge (5 mg Tablette), mg/Einheit | Menge (20 mg Tablette), mg/Einheit | Standard |
|---|---|---|---|
| Hydrokortison | 5, 0 | 20,0 | Ph.Eur. |
| Hypromellose K 100 cP | 47,05 | 41,2 | Ph.Eur. |
| (Methocel K 100) | | | |
| Hypromellose K 4000 | 20,0 | 24,6 | Ph.Eur. |
| cP (Methocel K4M) | | | |
| Mikrokristalline | 100,8 | 100,8 | Ph.Eur. |
| Cellulose (Avicel PH-102) | | | |
| Vorgelatinisierte Stärke (Stärke 1500) | 16,4 | 16,4 | Ph.Eur. |
| Hochdisperses Siliciumdioxid (Aerosil 200) | 1,0 | 1,0 | Ph.Eur. |
| Magnesiumstearat | 1,0 | 1,0 | Ph.Eur. |
| Opadry II | Ca. 13,75 | Ca. 11,0 | Colorcon |
| Gereinigtes Wasser* | Ca. 102 | Ca. 107 | Ph.Eur. |
und die orale Testdosis in Form einer Einzeleinheitsdosisform vorliegt, die einen Kern umfasst, der den Teil mit verlängerter Freisetzung enthält, wobei der Kern beschichtet ist, wobei die Beschichtung der Teil mit sofortiger Freisetzung ist; und im Fall einer 5-mg-Tablette eine Menge von 1,25 mg Hydrokortison in der Beschichtung vorliegt und 3,75 mg Hydrokortison im Kern vorliegt, und im Fall einer 20-mg-Tablette die Beschichtung eine Menge von 5 mg Hydrokortison aufweist und eine Menge von 15 mg Hydrokortison im Kern vorliegt.

14. Zusammensetzung zur Verwendung nach einem der Ansprüche 1-9, wobei die Zusammensetzung nach einem Gewichtsnomogramm verabreicht wird, wie folgt:
| Dosis (mg) | Körpergewicht (kg) |
|---|---|
| 15 | 55-59 |
| 20 | 60-69 |
| 25 | 70-79 |
| 30 | 80-84 |
| 35 | 85-89 |
| 40 | 90-100 |
und wobei die Dosis die zu verabreichende tägliche Gesamtdosis von Hydrokortison ist und das Körpergewicht das Körpergewicht des Patienten ist.

## Revendications

1. Composition pour utilisation en thérapie de substitution par glucocorticoïdes par administration orale de la composition une fois par jour, le matin, selon un nomogramme pharmacocinétique (PK), de la façon suivante :
| | C₀ₕ≤150 nM | 150<C₀ₕ≤250 nM | 250<C₀ₕ≤350 nM |
|---|---|---|---|
| C_{cortisol} (nM) | Dose (mg) | Dose (mg) | Dose (mg) |
| ≥198 | 15 | 10 | 5 |
| 197-161 | 20 | 10 | 5 |
| 160-141 | 25 | 15 | 10 |
| 140-115 | 30 | 15 | 10 |
| 114-97 | 35 | 15 | 10 |
| <97 | 40 | 15 | 10 |
où la dose est la dose quotidienne totale d' hydrocortisone administrée et où (C_{cortisol} (nM) est la différence de concentration sérique en cortisol entre 6 heures post-administration et la situation pré-administration, comme défini dans la présente invention ;
où la composition est une composition double qui comprend une partie à libération immédiate et une partie à libération prolongée, et où la composition est préparée en utilisant une composition selon le tableau ci-après
| | |
|---|---|
| Glucocorticoïde | 1,5 % en masse à 6,5 % en masse |
| Polymère, agent liant | 15 % en masse à 25 % en masse |
| Charge | 30 % en masse à 40 % en masse |
| Agent glissant | 0,1 % en masse à 0,5 % en masse |
| Lubrifiant | 0,1 % en masse à 0,5 % en masse |
| Système d'enrobage par pellicule | 1 % en masse à 5 % en masse |
| Solvant | 25 % en masse à 40 % en masse |

2. Composition pour utilisation selon l'une quelconque des revendications précédentes, où le rapport des glucocorticoïdes dans la composition entre la partie à libération immédiate et la partie à libération prolongée est inclus dans l'intervalle allant d'environ 1:1 à environ 0,01:1.

3. Composition pour utilisation selon l'une quelconque des revendications précédentes, où la composition est administrée sous la forme d'une dose orale, où entre environ 15 et environ 35 % en masse de la quantité totale des glucocorticoïdes sont libérés immédiatement à l'administration et la partie restante des glucocorticoïdes est libérée de façon modifiée sur une durée d'au moins environ 8 heures, par exemple au moins environ 12 heures.

4. Composition pour utilisation selon l'une quelconque des revendications précédentes, où le ou les glucocorticoïdes sont administrés sous la forme d'une forme galénique unitaire comprenant un noyau contenant la partie à libération prolongée des glucocorticoïdes, le noyau étant enrobé de la partie restante des glucocorticoïdes, et où l'enrobage est la partie à libération immédiate.

5. Composition pour utilisation selon l'une quelconque des revendications précédentes, où l'administration de la dose à raison d'une fois par jour est complétée d'une ou de plusieurs doses supplémentaires de glucocorticoïdes, et peut d'autre part se dérouler deux fois par jour, 3 fois par jour, 4 fois par jour, 5 fois par jour ou 6 fois par jour.

6. Composition pour utilisation selon la revendication 5, où l'administration est orale ou parentérale.

7. Composition pour utilisation selon l'une quelconque des revendications précédentes, où l'intervalle de temps entre deux administrations consécutives quelconques est par exemple d'environ 2 heures ou plus, tel que par exemple d'environ 3 heures ou plus, tel que par exemple d'environ 4 heures ou plus, tel que par exemple d'environ 5 heures ou plus, tel que par exemple d'environ 6 heures ou plus, tel que par exemple d'environ 7 heures ou plus, tel que par exemple d'environ 8 heures ou plus, tel que par exemple d'environ 9 heures ou plus, tel que par exemple d'environ 10 heures ou plus, tel que par exemple d'environ 11 heures ou plus, tel que par exemple d'environ 12 heures ou plus, indépendamment les unes des autres.

8. Composition pour utilisation selon l'une quelconque des revendications précédentes, où la dose quotidienne totale de glucocorticoïdes est par exemple d'environ 15 mg, d'environ 20 mg, d'environ 25 mg, d'environ 30 mg, d'environ 40 mg, d'environ 50 mg, d'environ 60 mg, d'environ 70 mg, d'environ 80 mg, d'environ 90 mg, d'environ 100 mg, d'environ 150 mg, ou d'environ 200 mg.

9. Composition pour utilisation selon l'une quelconque des revendications précédentes, où l'administration peut être n'importe quelle combinaison de doses unitaires différentes, telles que par exemple une combinaison de n'importe quel nombre de formes galéniques contenant une dose de 5 mg et/ou n'importe quel nombre de formes galéniques contenant une dose de 20 mg.

10. Composition pour utilisation selon l'une quelconque des revendications précédentes, où la composition est sous la forme d'une forme galénique solide incluant un comprimé ou une capsule.

11. Composition pour utilisation selon l'une quelconque des revendications précédentes, où la composition est conforme au tableau ci-après
| Composant | Quantité (comprimé de 5 mg), mg/unité | Quantité (comprimé 20 mg), mg/unité | Norme |
|---|---|---|---|
| Hydrocortisone | 5, 0 | 20,0 | Ph.Eur. |
| Hypromellose K 100 (Methocel K 100) | cP47,05 | 41,2 | Ph.Eur. |
| Hypromellose K 4000 (Methocel K4M) | cP20,0 | 24,6 | Ph.Eur. |
| Cellulose, microcristalli (Avicel PH-102) | ine100,8 | 100,8 | Ph.Eur. |
| Amidon prégélifié (Star 1500) | rch16,4 | 16,4 | Ph.Eur. |
| Silice colloïdale anhyc (Aerosil 200) | anhydre1,0 | 1,0 | Ph.Eur. |
| Stéarate de magnésium Opadry II | 1,0 environ | 1,0 environ | Ph.Eur. Colorcon |
| | 13,75 | 11, 0 | |
| Eau, purifiée* | environ 102 | environ 107 Ph.Eur. | |
| | | | |
|---|---|---|---|
| *S'évapore pendant le processus de fabrication | | | |
où la composition est sous la forme d'une forme galénique unitaire comprenant un noyau contenant la partie à libération prolongée, le noyau étant enrobé, où l'enrobage est la partie à libération immédiate ;
et dans le cas d'un comprimé de 5 mg, une quantité de 1,25 mg d'hydrocortisone se trouve dans l'enrobage et 3,75 mg d'hydrocortisone se trouvent dans le noyau, et dans le cas d'un comprimé de 20 mg, l'enrobage contient une quantité de 5 mg d'hydrocortisone et une quantité de 15 mg d'hydrocortisone se trouve dans le noyau.

12. Méthode de dérivation d'une posologie individualisée de glucocorticoïdes, la méthode comprenant :
(i) la détermination, par une méthode de dosage immunologique, de la concentration sérique en cortisol C₀ₕ d'un échantillon sanguin pré-administration, où l'échantillon sanguin pré-administration est un échantillon avant administration d'une dose de glucocorticoïde à un sujet dans un état à jeun,
(ii) la détermination, par une méthode de dosage immunologique, de la concentration sérique en cortisol C₆ₕ d'un échantillon sanguin post-administration, où l'échantillon sanguin post-administration est un échantillon prélevé à exactement 6 heures post-administration d'une dose d'hydrocortisone de 20 mg ou d'une dose d'essai oral d'équivalent d'hydrocortisone, et où la dose d'essai oral est la première dose d'hydrocortisone ou d'équivalent d'hydrocortisone ce jour spécifique,
(iii) la dérivation de la différence de concentration sérique en cortisol (C_{cortisol} = C₆ₕ-C₀ₕ) entre 6 heures post-administration (C₆ₕ) et la situation pré-administration (C₀ₕ),
(iv) la dérivation de la dose individuelle d'hydrocortisone ou d'équivalent d'hydrocortisone administrée une fois par jour à partir des valeurs de C_{cortisol} dérivée et de C₀ₕ et du nomogramme PK correspondant au tableau ci-après
| | C₀ₕ≤150 nM | 150<C₀ₕ≤5250 nM | 250<C₀ₕ ≤350 nM |
|---|---|---|---|
| C_{cortisol} (nM) | Dose (mg) | Dose (mg) | Dose (mg) |
| ≥198 | 15 | 10 | 5 |
| 197-161 | 20 | 10 | 5 |
| 160-141 | 25 | 15 | 10 |
| 140-115 | 30 | 15 | 10 |
| 114-97 | 35 | 15 | 10 |
| <97 | 40 | 15 | 10 |
où la dose d'essai oral est une composition double qui comprend une partie à libération immédiate et une partie à libération prolongée, et où la dose d'essai oral est préparée en utilisant une composition selon le tableau ci-après
| | |
|---|---|
| Glucocorticoïde | 1,5 % en masse à 6,5 % en masse |
| Polymère, agent liant | 15 % en masse à 25 % en masse |
| Charge | 30 % en masse à 40 % en masse |
| Agent glissant | 0,1 % en masse à 0,5 % en masse |
| Lubrifiant | 0,1 % en masse à 0,5 % en masse |
| Système d'enrobage par pellicule | 1 % en masse à 5 % en masse |
| Solvant | 25 % en masse à 40 % en masse |

13. Méthode selon la revendication 12, où la dose d'essai oral correspond au tableau ci-après
| Composant | Quantité (comprimé de 5 mg), mg/unité | Quantité (comprimé 20 mg), mg/unité | Norme |
|---|---|---|---|
| Hydrocortisone | 5, 0 | 20,0 | Ph.Eur. |
| Hypromellose K 100 cP (Methocel K 100) | 47,05 | 41,2 | Ph.Eur. |
| Hypromellose K 4000 cP (Methocel K4M) | 20,0 | 24,6 | Ph.Eur. |
| Cellulose, microcristalline (Avicel PH-102) | 100, 8 | 100,8 | Ph.Eur. |
| Amidon prégélifié (Starch 1500) | 16,4 | 16,4 | Ph.Eur. |
| Silice colloïdale anhydre (Aerosil 200) | 1,0 | 1,0 | Ph.Eur. |
| Stéarate de magnésium | 1,0 | 1,0 | Ph.Eur. |
| Opadry II | environ 13,75 | environ 11,0 | Colorcon |
| Eau, purifiée* | environ 102 | environ 107 | Ph.Eur. |
et la dose d'essai oral est sous la forme d'une forme galénique unitaire comprenant un noyau contenant la partie à libération prolongée, le noyau étant enrobé, où l'enrobage est la partie à libération immédiate ; et dans le cas d'un comprimé de 5 mg, une quantité de 1,25 mg d'hydrocortisone se trouve dans l'enrobage et 3,75 mg d'hydrocortisone se trouvent dans le noyau, et dans le cas d'un comprimé de 20 mg, l'enrobage inclut une quantité de 5 mg d'hydrocortisone et une quantité de 15 mg d'hydrocortisone se trouve dans le noyau.

14. Composition pour utilisation selon l'une quelconque des revendications 1 à 9, où la composition est administrée selon un nomogramme de poids, de la façon suivante :
| Dose (mg) | Poids corporel (kg) |
|---|---|
| 15 | 55-59 |
| 20 | 60-69 |
| 25 | 70-79 |
| 30 | 80-84 |
| 35 | 85-89 |
| 40 | 90-100 |
et où la dose est la dose quotidienne totale d'hydrocortisone à administrer et le poids corporel est le poids corporel du sujet.
